# EUROPEAN PATENT APPLICATION

(11) **EP 1 739 193 A2**
(43) Date of publication of application: **03.01.2007**
(21) Application number: 06450087.9
(22) Date of filing: 26.06.2006
(51) Int. Cl.: C12Q 1/68

(54) **Salmonella serotyping**

(30) Priority: 27.06.2005 AT 10762005
(71) Applicant: ARC Seibersdorf Research GmbH, 1010 Wien (AT)
(72) Inventor: Bodrossy, Levente, 9068 Töltéstava (HU); Sandjong, Bertrand Tankouo, G1X 3W8 Québec (CA); Sessitsch, Angela, 1140 Vienna (AT)
(74) Representative: Sonn & Partner Patentanwälte

(57) **Abstract**

The present invention provides a set of oligonucleotide molecules for Salmonella serotyping comprising oligonucleotide probes specific for at least one member of each of the four Salmonella genes selected from the *gyrB, atpD, fliC* and *fljB* genes, wherein each probe comprises at least one diagnostic region.

SEQUENCE LISTING

<110>  ARC Seibersdorf Research GmbH

<120>  Salmonella Serotyping

<130>  r47651

<150>  AT 1076/2005
<151>  2005-06-27

<160>  201

<170>  PatentIn version 3.3

<210>  1
<211>  27
<212>  DNA
<213>  Salmonella

<400>  1
caagacgact aaatctactg ctggtac                                          27


<210>  2
<211>  27
<212>  DNA
<213>  Salmonella

<400>  2
gtaccagcag tagatttagt cgtcttg                                          27


<210>  3
<211>  22
<212>  DNA
<213>  Salmonella

<400>  3
ggtgtggaag tagcgctaca gt                                               22


<210>  4
<211>  21
<212>  DNA
<213>  Salmonella

<400>  4
ttcaagttgg ggcgaatgac g                                                21


<210>  5
<211>  25
<212>  DNA
<213>  Salmonella

<400>  5
gtggtcccgc agcagataaa ttatc                                            25


<210>  6
<211>  22
<212>  DNA
<213>  Salmonella

<400>  6
tgccgccttc gtagtggaaa tg                                               22


<210>  7

<211>  19
<212>  DNA
<213>  Salmonella

<400>  7
cctgccgcat cgtctttcg                                                    19


<210>  8
<211>  22
<212>  DNA
<213>  Salmonella

<400>  8
caacaacaac ctgcagcgtg tg                                                22


<210>  9
<211>  22
<212>  DNA
<213>  Salmonella

<400>  9
ggacaacacc ctgaccatcc ag                                                22


<210>  10
<211>  21
<212>  DNA
<213>  Salmonella

<400>  10
aacaacaacc tgcagcgtgt g                                                 21


<210>  11
<211>  25
<212>  DNA
<213>  Salmonella

<400>  11
gcagttcctg agagtttgac aactc                                             25


<210>  12
<211>  25
<212>  DNA
<213>  Salmonella

<400>  12
ctgaacgaaa tcgaccgtgt atccg                                             25


<210>  13
<211>  20
<212>  DNA
<213>  Salmonella

<400>  13
aacggcgtga aagtcctggc                                                   20


<210>  14
<211>  21
<212>  DNA
<213>  Salmonella

<400>  14
gaactggcgg ttcagtctgc t                                                 21

<210>  15
<211>  18
<212>  DNA
<213>  Salmonella

<400>  15
cagcgcgcct tcagtggt                                                    18


<210>  16
<211>  18
<212>  DNA
<213>  Salmonella

<400>  16
cgcagcgcat ccacctgc                                                    18


<210>  17
<211>  20
<212>  DNA
<213>  Salmonella

<400>  17
ggaaatccca cggcaacagg                                                  20


<210>  18
<211>  17
<212>  DNA
<213>  Salmonella

<400>  18
ggctattcac cgcgcgg                                                     17


<210>  19
<211>  18
<212>  DNA
<213>  Salmonella

<400>  19
gggctattca ccgcgcgg                                                    18


<210>  20
<211>  20
<212>  DNA
<213>  Salmonella

<400>  20
gcgtcggacg gattttccag                                                  20


<210>  21
<211>  24
<212>  DNA
<213>  Salmonella

<400>  21
gtaccatcgt gccagtttta tcgg                                             24


<210>  22
<211>  26
<212>  DNA
<213>  Salmonella

<400>  22

tgtctttggt ctgcgaggaa aatttc                                          26

<210>  23
<211>  18
<212>  DNA
<213>  Salmonella

<400>  23
tgctttgcag atccgccc                                                  18

<210>  24
<211>  18
<212>  DNA
<213>  Salmonella

<400>  24
ggcacgagcg gcatcaat                                                  18

<210>  25
<211>  26
<212>  DNA
<213>  Salmonella

<400>  25
attgatcggc ttttaaatct gctcca                                          26

<210>  26
<211>  22
<212>  DNA
<213>  Salmonella

<400>  26
aaaccgttga cgcattccgt ag                                              22

<210>  27
<211>  22
<212>  DNA
<213>  Salmonella

<400>  27
ggcagtaccc tccgtagttt ta                                              22

<210>  28
<211>  22
<212>  DNA
<213>  Salmonella

<400>  28
cgcaacttca acaccgatac cg                                              22

<210>  29
<211>  27
<212>  DNA
<213>  Salmonella

<400>  29
ggaaaccatt tttacctgag tcagtcg                                         27

<210>  30
<211>  24
<212>  DNA
<213>  Salmonella

<400>  30
ctaagaattc cttaatccgg gcgg                                              24

<210>  31
<211>  18
<212>  DNA
<213>  Salmonella

<400>  31
cgttgcccgc cgatgcta                                                     18

<210>  32
<211>  18
<212>  DNA
<213>  Salmonella

<400>  32
gagtcccgcc atcacgct                                                     18

<210>  33
<211>  24
<212>  DNA
<213>  Salmonella

<400>  33
agcttatctt tggtctggga ggag                                              24

<210>  34
<211>  22
<212>  DNA
<213>  Salmonella

<400>  34
cgaactctac ctggtggaag gg                                                22

<210>  35
<211>  26
<212>  DNA
<213>  Salmonella

<400>  35
gtgatttcag cctagatgga gtcgag                                            26

<210>  36
<211>  24
<212>  DNA
<213>  Salmonella

<400>  36
gcgatagcta gtgccattaa gggt                                              24

<210>  37
<211>  19
<212>  DNA
<213>  Salmonella

<400>  37
actgccgatg caaatgccg                                                    19

<210>  38
<211>  24

<212>  DNA
<213>  Salmonella

<400>  38
ctggcgacgc tactaaaaat ggtg                                              24


<210>  39
<211>  20
<212>  DNA
<213>  Salmonella

<400>  39
ctaattaccg cgctgggctg                                                   20


<210>  40
<211>  22
<212>  DNA
<213>  Salmonella

<400>  40
accccaagtt ataccgctgc tg                                                22


<210>  41
<211>  24
<212>  DNA
<213>  Salmonella

<400>  41
gctttaagcg cataaccacc ttca                                              24


<210>  42
<211>  26
<212>  DNA
<213>  Salmonella


<220>
<221>  misc_feature
<222>  (5)..(5)
<223>  n is a, c, g, or t

<400>  42
ggttntagct ttaactgctc ctgtcg                                            26


<210>  43
<211>  18
<212>  DNA
<213>  Salmonella

<400>  43
ctgatgtgcc cgttcgcg                                                     18


<210>  44
<211>  18
<212>  DNA
<213>  Salmonella

<400>  44
ccggttcgcc cagaacgt                                                     18


<210>  45
<211>  25
<212>  DNA

<213>  Salmonella

<400>  45
aggtagaact gataacgtct gtcgc                                          25


<210>  46
<211>  22
<212>  DNA
<213>  Salmonella

<400>  46
cacgcgcggc atcgataata tt                                             22


<210>  47
<211>  23
<212>  DNA
<213>  Salmonella

<400>  47
agcagtgctg atacttccgt acc                                            23


<210>  48
<211>  26
<212>  DNA
<213>  Salmonella

<400>  48
atctaagccc agctcagttt tatcga                                         26


<210>  49
<211>  21
<212>  DNA
<213>  Salmonella

<400>  49
ccaagtctac ggcagtaccc t                                              21


<210>  50
<211>  26
<212>  DNA
<213>  Salmonella

<400>  50
gtgatgtaaa aattgcggca gctgat                                         26


<210>  51
<211>  21
<212>  DNA
<213>  Salmonella

<400>  51
cggatggaaa cgcctgagtt c                                              21


<210>  52
<211>  28
<212>  DNA
<213>  Salmonella


<220>
<221>  misc_feature
<222>  (2)..(2)
<223>  n is a, c, g, or t

<400>  52
cnatagtaac aacctcggtt ttaccgtc                                          28

<210>  53
<211>  26
<212>  DNA
<213>  Salmonella

<400>  53
tgatttcgga tccattgcat cagttg                                            26

<210>  54
<211>  22
<212>  DNA
<213>  Salmonella

<400>  54
cgaggatgtt ggagacttcg gt                                                22

<210>  55
<211>  23
<212>  DNA
<213>  Salmonella

<400>  55
ggatacgctg aacgtgcaga aag                                               23

<210>  56
<211>  18
<212>  DNA
<213>  Salmonella

<400>  56
aggttggcgc gaacgacg                                                     18

<210>  57
<211>  23
<212>  DNA
<213>  Salmonella

<400>  57
acaactcttc gtaagaaggc gct                                               23

<210>  58
<211>  22
<212>  DNA
<213>  Salmonella

<400>  58
cggctgataa cctactgcgg aa                                                22

<210>  59
<211>  23
<212>  DNA
<213>  Salmonella

<400>  59
cgaacagcag tacatcacga cct                                               23

<210>  60
<211>  23

<212>  DNA
<213>  Salmonella

<400>  60
gttggtaaag gtttcgtggc tcg                                              23


<210>  61
<211>  25
<212>  DNA
<213>  Salmonella

<400>  61
caaaacttcc atgcctaagc gaact                                            25


<210>  62
<211>  24
<212>  DNA
<213>  Salmonella

<400>  62
cgatttctga ctgtgttagc ctgg                                             24


<210>  63
<211>  23
<212>  DNA
<213>  Salmonella

<400>  63
cccaaagcgt aaccatgtgt gaa                                              23


<210>  64
<211>  24
<212>  DNA
<213>  Salmonella

<400>  64
gttattgtgt agccagaatg ccgc                                             24


<210>  65
<211>  25
<212>  DNA
<213>  Salmonella

<400>  65
aagcgaactg ttgagagtac gtttc                                            25


<210>  66
<211>  22
<212>  DNA
<213>  Salmonella

<400>  66
ggaagtgctt gtcccaacct tg                                               22


<210>  67
<211>  25
<212>  DNA
<213>  Salmonella

<400>  67
aattttgagc tagtagggtt gcagc                                            25

<210> 68
<211> 24
<212> DNA
<213> Salmonella

<400> 68
cagccacgag taagtcttcc cttg 24

<210> 69
<211> 25
<212> DNA
<213> Salmonella

<400> 69
gagagatccc ctcataattt cccca 25

<210> 70
<211> 22
<212> DNA
<213> Salmonella

<400> 70
gacggcacta gcaaaacagc ag 22

<210> 71
<211> 26
<212> DNA
<213> Salmonella

<400> 71
cgtcagcagc agtataactt gtggtt 26

<210> 72
<211> 24
<212> DNA
<213> Salmonella

<400> 72
ttaattgctc ctgtcgcttc atcg 24

<210> 73
<211> 26
<212> DNA
<213> Salmonella

<400> 73
gtaaaacaat tgaaggcggt tatgcg 26

<210> 74
<211> 23
<212> DNA
<213> Salmonella

<400> 74
gttggttagc cgctgttttg gta 23

<210> 75
<211> 24
<212> DNA
<213> Salmonella

<400> 75
aaagttgtga ccttcagctt tgct 24

<210>  76
<211>  24
<212>  DNA
<213>  Salmonella

<400>  76
acaactcttc ataggaaggc gctg                                              24


<210>  77
<211>  18
<212>  DNA
<213>  Salmonella

<400>  77
tcagcgtggc cacttcct                                                     18


<210>  78
<211>  23
<212>  DNA
<213>  Salmonella

<400>  78
gatcgatttc ctccagacgt tgc                                               23


<210>  79
<211>  23
<212>  DNA
<213>  Salmonella

<400>  79
cgtaaccgta aaaaccaggc gat                                               23


<210>  80
<211>  23
<212>  DNA
<213>  Salmonella

<400>  80
ttcatatcga ccggttcacc cag                                               23


<210>  81
<211>  21
<212>  DNA
<213>  Salmonella

<400>  81
gccagcgcag catcaatttt c                                                 21


<210>  82
<211>  21
<212>  DNA
<213>  Salmonella

<400>  82
cggatcgcgt tcctgacaat c                                                 21


<210>  83
<211>  20
<212>  DNA
<213>  Salmonella

<400>  83
aaaattatcg acgccgcccg                                                    20

<210>  84
<211>  23
<212>  DNA
<213>  Salmonella

<400>  84
ggctttactc gcagcgtaag ttt                                                23

<210>  85
<211>  22
<212>  DNA
<213>  Salmonella

<400>  85
gaatctgcgc acgagacatg tt                                                 22

<210>  86
<211>  20
<212>  DNA
<213>  Salmonella

<400>  86
ccgcgccaat ggcaatatca                                                    20

<210>  87
<211>  25
<212>  DNA
<213>  Salmonella

<400>  87
ctttatccgg tgctactgca tcagt                                              25

<210>  88
<211>  22
<212>  DNA
<213>  Salmonella

<400>  88
ctgtagtgac tgatgcagca gc                                                 22

<210>  89
<211>  22
<212>  DNA
<213>  Salmonella

<400>  89
tccagcaagt attcgctcag ca                                                 22

<210>  90
<211>  21
<212>  DNA
<213>  Salmonella

<400>  90
acgccacggg tgataagatc a                                                  21

<210>  91
<211>  25
<212>  DNA

<213>  Salmonella

<400>  91
gacctttaat gttcgcggtg aaacg                                          25


<210>  92
<211>  18
<212>  DNA
<213>  Salmonella

<400>  92
tcaccgacct ccagcacc                                                  18


<210>  93
<211>  18
<212>  DNA
<213>  Salmonella

<400>  93
ctccaacatg tcccgcgc                                                  18


<210>  94
<211>  22
<212>  DNA
<213>  Salmonella

<400>  94
tcttcaccga tgtcgccttt ca                                             22


<210>  95
<211>  24
<212>  DNA
<213>  Salmonella

<400>  95
ggctttacct accggaactt cgat                                           24


<210>  96
<211>  26
<212>  DNA
<213>  Salmonella

<400>  96
gctgtagcct tctttgtcca tatagg                                         26


<210>  97
<211>  27
<212>  DNA
<213>  Salmonella

<400>  97
accaactgca actgatatta aagctgc                                        27


<210>  98
<211>  24
<212>  DNA
<213>  Salmonella

<400>  98
acaacttcag ttttaccgtc tgcg                                           24


<210>  99

<211>  23
<212>  DNA
<213>  Salmonella

<400>  99
cgggttttcg gtagttgtag cag                                              23


<210>  100
<211>  24
<212>  DNA
<213>  Salmonella

<400>  100
ggtgatgact actatgctgc aact                                             24


<210>  101
<211>  24
<212>  DNA
<213>  Salmonella

<400>  101
ggaagtgata gaaccggtct tggt                                             24


<210>  102
<211>  18
<212>  DNA
<213>  Salmonella

<400>  102
cggtacccag tgcggact                                                    18


<210>  103
<211>  22
<212>  DNA
<213>  Salmonella

<400>  103
cctggatggt caaggtgttg tc                                               22


<210>  104
<211>  24
<212>  DNA
<213>  Salmonella

<400>  104
ttcatctgac catacaccag ggat                                             24


<210>  105
<211>  22
<212>  DNA
<213>  Salmonella

<400>  105
cggtatcgta cgtaccatcg cg                                               22


<210>  106
<211>  20
<212>  DNA
<213>  Salmonella

<400>  106
gcaaacggct cgaactctgg                                                  20

<210>  107
<211>  23
<212>  DNA
<213>  Salmonella

<400>  107
aaatcagttt gttccgcgac acg                                              23


<210>  108
<211>  24
<212>  DNA
<213>  Salmonella

<400>  108
gtggaaatga tcctctttgc cgtc                                             24


<210>  109
<211>  26
<212>  DNA
<213>  Salmonella

<400>  109
tgaaagttgc attcccgtct ttatct                                           26


<210>  110
<211>  18
<212>  DNA
<213>  Salmonella

<400>  110
acgcgccttc agtcgtct                                                    18


<210>  111
<211>  23
<212>  DNA
<213>  Salmonella

<400>  111
gcgcatcatc aatttttgcc agc                                              23


<210>  112
<211>  22
<212>  DNA
<213>  Salmonella

<400>  112
cgccaacgct gcatcaattt tc                                               22


<210>  113
<211>  23
<212>  DNA
<213>  Salmonella

<400>  113
tgttggagac ttcggtcgca tag                                              23


<210>  114
<211>  20
<212>  DNA
<213>  Salmonella

<400>  114

cgaaggtcgt gacgtcctgc                                                20

<210>  115
<211>  18
<212>  DNA
<213>  Salmonella

<400>  115
gctctgcgaa gcagggcc                                                  18

<210>  116
<211>  22
<212>  DNA
<213>  Salmonella

<400>  116
tacttccgta ccagcaaggg tg                                             22

<210>  117
<211>  25
<212>  DNA
<213>  Salmonella

<400>  117
tttctgcaca ttcagtgaat ccagg                                          25

<210>  118
<211>  23
<212>  DNA
<213>  Salmonella

<400>  118
ccggttttgg ttgaggtgat acg                                            23

<210>  119
<211>  18
<212>  DNA
<213>  Salmonella

<400>  119
cagcgctact tccacacc                                                  18

<210>  120
<211>  18
<212>  DNA
<213>  Salmonella

<400>  120
cagccacttc cgccagct                                                  18

<210>  121
<211>  21
<212>  DNA
<213>  Salmonella

<400>  121
gcagaccaga agacagacgc t                                              21

<210>  122
<211>  25
<212>  DNA
<213>  Salmonella

<400>  122
caatcgtagt atcggcatat cctgt                                          25

<210>  123
<211>  24
<212>  DNA
<213>  Salmonella

<400>  123
gctactggtc ttggtggtac tgac                                           24

<210>  124
<211>  22
<212>  DNA
<213>  Salmonella

<400>  124
ttaagcctcg gctactggtc tt                                             22

<210>  125
<211>  26
<212>  DNA
<213>  Salmonella

<400>  125
ctttgctggc attgtatgtt ttaccg                                         26

<210>  126
<211>  18
<212>  DNA
<213>  Salmonella

<400>  126
gtagcagccg cttccgcc                                                  18

<210>  127
<211>  19
<212>  DNA
<213>  Salmonella

<400>  127
tccgcggtag gctatcagc                                                 19

<210>  128
<211>  19
<212>  DNA
<213>  Salmonella

<400>  128
gctttggcgc aggttgatg                                                 19

<210>  129
<211>  21
<212>  DNA
<213>  Salmonella

<400>  129
ctgcagcggg ttttcagtct t                                              21

<210>  130
<211>  21

<212>  DNA
<213>  Salmonella

<400>  130
ggtctggcca gaaacacggt c                                                21


<210>  131
<211>  21
<212>  DNA
<213>  Salmonella

<400>  131
gctacgggct gaagacaggt t                                                21


<210>  132
<211>  19
<212>  DNA
<213>  Salmonella

<400>  132
gccagcgggt tttcagtgg                                                   19


<210>  133
<211>  20
<212>  DNA
<213>  Salmonella

<400>  133
ggttttaccc acacccgcac                                                  20


<210>  134
<211>  25
<212>  DNA
<213>  Salmonella

<400>  134
tgtaggcatc ctggacatta agctt                                            25


<210>  135
<211>  19
<212>  DNA
<213>  Salmonella

<400>  135
agacggatgg agacgcctg                                                   19


<210>  136
<211>  21
<212>  DNA
<213>  Salmonella

<400>  136
aacaacctgt cttctgcccg t                                                21


<210>  137
<211>  27
<212>  DNA
<213>  Salmonella

<400>  137
cagaccttta atgttcgcgg taaaacg                                          27

<210> 138
<211> 25
<212> DNA
<213> Salmonella

<400> 138
gcgcaataga gataccgtcg ttagc 25

<210> 139
<211> 18
<212> DNA
<213> Salmonella

<400> 139
ggatgcaaat gccgcgac 18

<210> 140
<211> 18
<212> DNA
<213> Salmonella

<400> 140
gcggcaggtc aggcaatt 18

<210> 141
<211> 23
<212> DNA
<213> Salmonella

<400> 141
gctgcatagg tgtcataacc cgt 23

<210> 142
<211> 23
<212> DNA
<213> Salmonella

<400> 142
cagcgggtgc caataaatat cgt 23

<210> 143
<211> 19
<212> DNA
<213> Salmonella

<400> 143
gtaaagctac gctgggccg 19

<210> 144
<211> 26
<212> DNA
<213> Salmonella

<400> 144
gaagtgaatg ttgatagtgc gacagg 26

<210> 145
<211> 26
<212> DNA
<213> Salmonella

<400> 145
tcagatctat atcgatggtt tcaccg 26

<210>  146
<211>  18
<212>  DNA
<213>  Salmonella

<400>  146
acgaaccgcc gggaaacc                                                    18


<210>  147
<211>  25
<212>  DNA
<213>  Salmonella

<400>  147
ggctatcgca acttctatca aaggc                                            25


<210>  148
<211>  26
<212>  DNA
<213>  Salmonella

<400>  148
ttctaatatg tccaaagcgc agatcc                                           26


<210>  149
<211>  24
<212>  DNA
<213>  Salmonella

<400>  149
tgatacgacc caacgtggct ttac                                             24


<210>  150
<211>  19
<212>  DNA
<213>  Salmonella

<400>  150
caccgaaaac ccactggct                                                   19


<210>  151
<211>  25
<212>  DNA
<213>  Salmonella

<400>  151
kgkwaagtct tcttcgatgc actga                                            25


<210>  152
<211>  24
<212>  DNA
<213>  Salmonella

<400>  152
accagaagac agacgctcaa tagc                                             24


<210>  153
<211>  26
<212>  DNA
<213>  Salmonella

<400>  153
ttgcttcagc agatgctaaa aatgcc                                          26

<210>  154
<211>  21
<212>  DNA
<213>  Salmonella

<400>  154
ccgatgctaa tgccgctaca t                                               21

<210>  155
<211>  24
<212>  DNA
<213>  Salmonella

<400>  155
tcgggtctta ctgatgcagc tatt                                            24

<210>  156
<211>  24
<212>  DNA
<213>  Salmonella

<400>  156
atcggcataa gctgtcgttg ttac                                            24

<210>  157
<211>  20
<212>  DNA
<213>  Salmonella

<400>  157
cgtaccagtc gtaccacccg                                                 20

<210>  158
<211>  19
<212>  DNA
<213>  Salmonella

<400>  158
cgtgaaggta ccggacccg                                                  19

<210>  159
<211>  23
<212>  DNA
<213>  Salmonella

<400>  159
gcggctttga tagcagtagc atc                                             23

<210>  160
<211>  18
<212>  DNA
<213>  Salmonella

<400>  160
ggcatcatcg ccagtggc                                                   18

<210>  161
<211>  18
<212>  DNA

<213>  Salmonella

<400>  161
ggcatcatcg ccagtggc                                                    18


<210>  162
<211>  27
<212>  DNA
<213>  Salmonella

<400>  162
ggtggagaag tagaatatat tcgggtg                                          27


<210>  163
<211>  25
<212>  DNA
<213>  Salmonella

<400>  163
gttttatcgg tatcgccagt aacgg                                            25


<210>  164
<211>  21
<212>  DNA
<213>  Salmonella

<400>  164
ccacgatttt cgcgtcagac g                                                21


<210>  165
<211>  23
<212>  DNA
<213>  Salmonella

<400>  165
tggatactgc agggacttac tgg                                              23


<210>  166
<211>  22
<212>  DNA
<213>  Salmonella

<400>  166
gcaaagctgt gaccggtgtt tc                                               22


<210>  167
<211>  23
<212>  DNA
<213>  Salmonella

<400>  167
gcataacctc cgtcaatcgt ctt                                              23


<210>  168
<211>  27
<212>  DNA
<213>  Salmonella

<400>  168
cccagaaaaa agatggtagc tttagtg                                          27


<210>  169

<211>  22
<212>  DNA
<213>  Salmonella

<400>  169
ggcagaatcg cctgattctt gc                                                22


<210>  170
<211>  18
<212>  DNA
<213>  Salmonella

<400>  170
atttcacggg cacgacgc                                                     18


<210>  171
<211>  23
<212>  DNA
<213>  Salmonella

<400>  171
aataccacgt cttacaccgc aac                                               23


<210>  172
<211>  26
<212>  DNA
<213>  Salmonella

<400>  172
gctatctaca tcatacgctt tctgca                                            26


<210>  173
<211>  25
<212>  DNA
<213>  Salmonella

<400>  173
cccagttggt tcagtgctat tttgg                                             25


<210>  174
<211>  23
<212>  DNA
<213>  Salmonella

<400>  174
ctggatagtc agggtgttgt cct                                               23


<210>  175
<211>  25
<212>  DNA
<213>  Salmonella

<400>  175
tctgggctgt aacagtatct gtacc                                             25


<210>  176
<211>  25
<212>  DNA
<213>  Salmonella

<400>  176
ccccagtaaa agcccccaat agtaa                                             25

<210>  177
<211>  20
<212>  DNA
<213>  Salmonella

<400>  177
tcagcgctcc ttcagtggtc                                                    20


<210>  178
<211>  26
<212>  DNA
<213>  Salmonella

<400>  178
tactgatatc gaaactgcaa ttggcg                                             26


<210>  179
<211>  26
<212>  DNA
<213>  Salmonella

<400>  179
agttgtttcc gcagatgcta aaaatg                                             26


<210>  180
<211>  24
<212>  DNA
<213>  Salmonella

<400>  180
tcggtcgcat agtcagaatc ttcg                                               24


<210>  181
<211>  23
<212>  DNA
<213>  Salmonella

<400>  181
atctcgcctt tcatgtcaac cgg                                                23


<210>  182
<211>  24
<212>  DNA
<213>  Salmonella

<400>  182
atcttcttta ccatcgcgct tgtc                                               24


<210>  183
<211>  20
<212>  DNA
<213>  Salmonella

<400>  183
ggttttaccc acacccgcac                                                    20


<210>  184
<211>  21
<212>  DNA
<213>  Salmonella

<400>  184

aagacgattg atggcggctt c                                                21

<210>  185
<211>  26
<212>  DNA
<213>  Salmonella

<400>  185
ggcgttaatg ctgatcgatc catctt                                           26

<210>  186
<211>  27
<212>  DNA
<213>  Salmonella

<400>  186
gttggagacg atatttatgc tgcaact                                          27

<210>  187
<211>  26
<212>  DNA
<213>  Salmonella

<400>  187
gttggttcag tgcagttttt gtgttg                                           26

<210>  188
<211>  27
<212>  DNA
<213>  Salmonella

<400>  188
cggtgtattt agtccctgaa cttcagt                                          27

<210>  189
<211>  21
<212>  DNA
<213>  Salmonella

<400>  189
cgacttcccc gcttacaggt g                                                21

<210>  190
<211>  24
<212>  DNA
<213>  Salmonella

<400>  190
tggtctgtga ggaaaatttc gggt                                             24

<210>  191
<211>  26
<212>  DNA
<213>  Salmonella

<400>  191
ctgggtaatt tcagcctgga tagagt                                           26

<210>  192
<211>  22
<212>  DNA
<213>  Salmonella

<400>  192
ggtttttacg gttacgcccc tg                                                22

<210>  193
<211>  23
<212>  DNA
<213>  Salmonella

<400>  193
ggccatacac cagggagact tta                                               23

<210>  194
<211>  20
<212>  DNA
<213>  Salmonella

<400>  194
tagttgacgt cgaattccct                                                   20

<210>  195
<211>  23
<212>  DNA
<213>  Salmonella

<400>  195
ggagacgggt cagtcaagtc atc                                               23

<210>  196
<211>  29
<212>  DNA
<213>  Salmonella

<400>  196
caagtcatta atacacaaca gcctgtcgc                                         29

<210>  197
<211>  28
<212>  DNA
<213>  Salmonella

<400>  197
ttaacgcagt aaagagagga cgttttgc                                          28

<210>  198
<211>  29
<212>  DNA
<213>  Salmonella

<400>  198
ggcacaagta atcaacacta acagtctgt                                         29

<210>  199
<211>  27
<212>  DNA
<213>  Salmonella

<400>  199
cgtaacagag acagcacgtt cttgctg                                           27

<210>  200
<211>  47

<212>  DNA
<213>  Salmonella

<400>  200
gaagtcatca tgaccgttct gcactgcacg tggacgtgga cgtaaag                47

<210>  201
<211>  56
<212>  DNA
<213>  Salmonella

<400>  201
agcagggtac ggatgtgcga gccagtcacg tacagtcacg tgcagtcacg tgtcat      56

## Description

The present invention relates to the field of bacterial identification methods, in particular genetic methods usable for the identification of characteristic genetic elements preferably using microarray techniques.

Salmonellae are bacteria and the etiologic agents of different diseases collectively referred to as salmonellosis. Human salmonellosis can be divided into four syndromes: enteric fever (typhoid-like disease), gastroenteritis, bacteremia with or without gastroenteritis, and the asymptomatic carrier state. To curb both typhoidal and nontyphoidal salmonellae, laboratory-based surveillance of human and animal infections is a necessary step of prevention strategy. Phenotypic methods play an important role in the identification to the genus level. Serotyping is used for primary typing of strains. However, serotyping of Salmonella is laborious due to the large number of recognized serotypes.

The genus *Salmonella* is subdivided into species *S. enterica,* which includes subspecies I (*S. e. enterica*)*,* II *(S. e. salamae),* IIIa (*S. e. arizonae*)*,* IIIb (*S. e. diarizonae*)*,* IV (*S. e*. *houtenae*) and VI (*S. e. indica*)*,* and *S. bongori* (formerly subspecies V). These species and subspecies are further divided into more than 2,500 serotypes.

*Salmonella* is a large genus, and serotyping is widely used to divide strains into groups according to their surface antigen variability. Serotyping is based on the immunological classification of the lipopolysaccharide moieties (O antigen), the flagellar protein (H antigen), and the capsular polysaccharide (Vi antigen). A unique feature of *Salmonella* among the *Enterobacteriaceae* is that this genus commonly has two distinct H antigens, the phase 1 and phase 2 flagellar antigens, which are regulated in a way, that only one antigen is expressed at a time. Generally, the Kauffmann-White scheme is used for the classification of *Salmonella* serotypes, which recognizes 46 O serogroups and 114 H antigens resulting in 2,523 characterized serotypes. Several antigenic factors may occur in one flagellum and therefore H antigens may be composed of various antigenic factors (e.g. H:e,n,x designates an antigen, which is composed of the factors e, n and x). Immunologically related flagellar antigens are grouped into complexes.

Serotyping represents a reliable and well-established methodology for the characterization of *Salmonella* strains, but is rather time-consuming and also requires a high number (167) of specific antisera. Furthermore, serotyping provides limited information on the genetic relationships of serovars and is not sufficient for making disease associations (Beltran et al., PNAS 85: 7753-7757 (1988)). Therefore, alternative, mainly DNA-based methodologies for the identification of *Salmonella* serotypes are being developed in several laboratories (Eicheta et al., Res Mic 153: 107-113 (2002); Nair et al., J Clin Mic 40(7): 2346-2351 (2002); Herrera-Leon et al., J Clin Mic 42(6); 2581-2586 (2004)). These novel techniques aim for higher specificity, better reproducibility as well as higher sensitivity, and mainly include multiplex PCR based detection and single-strand conformation polymorphism analysis. In addition, diagnostic microarrays, which allow high through-put, parallel detection and identification of a large number of microbes, represent a dynamically developing novel technical field. They have been mainly developed for environmental applications (e.g. Loy et al., App Env Mic 68(10): 5064-5081 (2002); Bodrossy et al., Env Mic 5(7): 566-582 (2003); Stralis-Pavese et al., Env Mic 6(4): 347-363 (2004)), but diagnostic microarrays have enormous potential for efficient high throughput analysis of pathogens (Bodrossy and Sessitsch, Curr Op Mic 7:245-254 (2004)). For the development of reliable *Salmonella* serotype - specific probes a thorough phylogenetic analysis of this genus and the identification of suitable markers are still required.

The O antigen is determined by the concerted activity of a varying number of enzymes, encoded by the rfb cluster. Thus, most of the *Salmonella* O antigen variation is a consequence of the genetic diversity within this gene cluster (Fitzgerald et al , App Env Mic 69(10): 6099-6105 (2003)). The microarray approach presented therein is not suitable for the analysis of presence/absence of a higher number of genes, thus there is a need for an alternative method to assess the O serogroup of *Salmonella.* As diversity in the O serogroup status (O antigen) is determined by the presence/absence of genes, rather than by point mutations, it is likely to be linked to the phylogenetic position of the analysed serovar.

Phylogenetic relationships of prokaryotes are most commonly based on the variability in their ribosomal RNA genes. However, for the classification of closely related species and subspecies rRNA genes are too conserved and alternative markers are required. As an alternative, the *gyrB* gene encoding the subunit B protein of DNA gyrase (topoisomerase type II) has been applied for the classification of bacteria including members of the *Enterobacteriaceae* (Fukushima et al., J Clin Mic 40(8): 2779-2785 (2002); Dauga, Int J Sys Evo Mic 52: 531-547 (2002)). Several studies demonstrated that the rate of molecular evolution of the *gyrB* gene is higher than that of ribosomal RNA genes (Dauga et al., 2002, above) and *gyrB* nucleotide sequences have been used to differentiate related species or strains of *Acinetobacter, Pseudomonas,* and *Shewanella.* Dauga (2002, above) described the *gyrB* sequence as useful to study intrageneric relationships of the *Enterobacteriaceae,* whereas the 16S rRNA gene sequence was found to be suitable to describe phylogenetic relationships between distantly related family members. The *atpD* gene, encoding the beta-subunit of bacterial F1F0 type ATP-synthases, has also been shown to reflect the phylogenetic position of bacteria. Its phylogenetic resolution power seems to be similar to that of the *gyrB* gene.

Two genes encode the flagellar antigens. The *fliC* gene that encodes the phase 1 (HI) antigen is located in one of the flagellar biosynthesis operons, is present in all *Salmonellae,* and has a homologue in *Escherichia coli.* The *fljB* gene encoding the phase 2 (H2) antigen is located in a region of the genome that is unique to *Salmonella* and is present in four of the six subspecies. Generally, the central part of flagellin genes is quite variable and is assumed to determine the epitope of the H antigen, whereas the 5' and 3' ends are typically conserved. McQuiston et al. (J Clin Mic 42(5): 1923-1932 (2004)) suggested the use of flagellin genes as useful targets for the molecular determination of the flagellar antigen type. In addition, a multiplex PCR system based on *fliC* gene variability has been established for distinguishing the most common phase-1 flagellar antigens (Herrera-Leon et al., J Clin Mic: 42(6): 2581-2586 (2004)).

DNA microarrays are a powerful tool for the parallel, high-throughput detection and quantification of many genes. Originally developed for whole genome gene expression analyses, DNA microarrays have a very strong application potential in many areas of microbiology. Upon availability of corresponding probe sets, they enable the detection of up to several thousand microbial strains, species or groups of species (depending on the design of the probe) in a single assay. In clinical, veterinary and plant microbiology, food and water quality control, this means that a single test can be developed to detect all pathogenic/beneficial/contaminating bacteria which might be present in the investigated sample. The potential for environmental microbiology is even stronger. By applying nested sets of oligonucleotide probes which target genes reflecting the phylogeny of the carrying organism, it becomes possible to roughly assess the whole prokaryotic diversity of an environment. The most obvious target for such studies is the 16S rRNA gene as a primary determinant for a species, and, for strain-specific identification, the intergenic spacer (IGS) region between the 16S rRNA and the 23S rRNA genes. Other - often referred to as "functional", i.e. encoding for related enzymes carrying out a defined function - possible target genes can also be used at least for a physiologically restricted group of microorganisms. The application of functional genes narrows down the analysis to a functionally (sometimes also phylogenetically) defined group of microbes. The main advantage of this approach is that it also enables the detection and analysis of uncultivated members of the investigated microbial groups.

Oligonucleotide probe sets spotted onto nylon or nitrocellulose membranes ("macroarrays") have been used for the diagnosis of bacteremia, food-contamination, detection of enterococci or cyanobacteria.

Microbial community structures can be assessed by the quantitative analysis of micro- or macroarray results. A simple and elegant method to quantify specific microbial genes from a sample applies gene fragments (500-900 bp in length) as probes. Each macroarray spot can consist of a mixture of an individual probe and a common reference gene fragment. Hybridization can be done with a Cy3 labeled environmental mixture and a Cy5 labeled reference DNA. With the Cy3/Cy5 marker system quantification is based on the Cy3/Cy5 ratios. Unfortunately, the same principle cannot be applied to the quantification of oligonucleotide chip results due to the inherent differences in hybridization efficiencies between oligonucleotide probes. In practice, one should design a different reference oligo for each probe.

The CN 1396270 describes a DNA microarray for detecting the frequently encountered pathogenic bacteria in water, such as Escherichia's bacteria, salmonella, staphylococcus, etc., which uses the 16S rRNA gene, two primers designed in the preservation region of 16S rRNA gene, and a probe for the variable region of 16s rRNA gene.

The DE 199 45 916 describes several oligonucleotide sequences for bacterial 23S/5S rRNA probes, which can be used in assays to distinguish bacteria of different categories. The serotypological determination of Salomonella is not disclosed therein.

In the US 6,087,104 the identification of several Bacillus species by PCR is disclosed. The provided PCR primers are specific for a 365 bp gyrB gene fragment which is not present in other bacteria and microorganisms which have a gyrB gene.

The US 2002/0146697 presents primers for the identification of micororganisms which are specific for the gyrB gene but do not bind the parE gene, a homologous gene of the gyrB gene.

The CA 2 307 010 provides a multitude of primer for the identification of microorganisms, in particular primers specific for antibiotic resistance genes and toxin genes.

The WO 1999/061458 provides nucleotide sequences to identify E. coli bacteria via their flagellin gene (fliC).

The EP 409 159 concerns the detection of bacteria which are responsobel for food poisoning, including Salmonella typhimurium, by PCR using specific primers, which are in the case of Salmonella typhimurium specific for the flagellin gene.

The aim of the present invention is therefore to provide a fast and efficient method for analysing in vitro and/or in vivo Salmonella serotypes (also referred to as serovars). An efficient method should also be capable to determine a plurality of serotypes (especially all or almost all clinically relevant strains) in a reliable, easily usable and standardized assay, preferably without prior bacteria isolation. A further objective of the present invention is to perform a DNA-based phylogenetic analysis of over 200 *Salmonella* strains belonging to the most prevalent 44 serovars.

Therefore, the present invention provides a set of oligonucleotide molecules for Salmonella serotyping, characterized in that at least one oligonucleotide molecule is specific for the *gyrB* gene, at least one oligonucleotide molecule is specific for the *atpD* gene, at least one oligonucleotide molecule is specific for the *fliC* gene and at least one oligonucleotide molecule is specific for the *fljB* gene. It has been surprisingly found that it is possible to provide a set of oligonucleotide molecules which are specific for the vast amount of Salmonella serotypes which, however, can be used all at once in the same hybridization step under the same hybridization conditions without preisolating different serotypes (e.g. 2-3-4 different Salmonella serotypes can be present in the original sample). The present invention provides several oligonucleotide molecules or probes, for serotyping. In certain cases it is possible to identify a sertotype with only a single oligonucleotide. However in order to identify a broader spectrum of Salmonella serotypes, oligonucleotides from each of the *gyrB, atpD, fliC, fljB* genes are provided. The combination of these genes is perfectly suitable for this task since certain serovars show significant differences in these genes. Accordingly, a minimal set consists of 4 oligos.

For a given application a set of oligonucleotide molecules (also referred to as oligonucleotides) can be selected from the group of possible oligonucleotide molecules given in table 1 below. However, different methodologies may require different criteria, thus the alteration of the probes may lead to different probes functioning perfectly under such different conditions. In an example of a serotyping procedure the set according to the invention is preferably applied to a PCR pre-enriched product of these 4 Salmonella genes, i.e. the genetic sequences are amplified (e.g. using the primers given in table 2 below) and the resulting PCR products are used for the serotyping with the set of the present invention. Another use for the set according to the invention is the use as PCR-primers. In the latter case serotyping would be based on the existence/non-existence of (or significantly weak) PCR products. Oligonucleotide molecules according to the invention are preferably selected from ribose or deoxyribose nucleotides.

The set according to the invention can be selected from oligonucleotides which are specific for the given Salmonella serovars, as long as specific nucleotides for each Salmonella gene selected from *gyrB, atpD, fliC* and *fljB* are included. The genetic sequences of these genes or the differences in these genes are known in the state of the art. Methods for oligonucleotide designs are given below in the examples (especially example 7) and include programs for the calculation of favourable melting points and hybridization models. The synthesis of oligonucleotides is nowadays a common procedure known by the artisan and can also be automated.

The term "oligonucleotide molecules" in the scope of the present application relates to any kind of oligonucleotide molecules, preferably DNA or RNA molecules, comprising a length which is efficient to be specific for a given serotype. This length, however, shall not extend over a maximum in order that the molecules are able to be used in high-throughput analysis without steric hindrance.

Preferably the set according to the invention is characterized in that the oligonucleotide molecules are specific for up to 30 nucleotide long regions of the gyrB gene, the atpD gene, the fliC gene or the fljB gene. Specificity for these short regions or diagnostic regions is commonly sufficient for an efficient hybridization procedure. Of course the length of the diagnostic regions and thus the length of the specific oligonucleotide molecules correlate and can be adjusted for certain serovar specifics. Especially preferred diagnostic regions are those related to the oligonucleotide probes given in table 1 below.

Another preferred set according to the invention comprises oligonucleotide molecules, which correspond to genetic sequences of the gyrB gene, the atpD gene, the fliC gene or the fljB gene located at the positions of these genes given for SEQ ID NOs 1 to 193 (given in table 1 below). At these positions variation between selected serovars exist, which can be used for serotyping with a set according to the invention.

Even more preferred the set according to the invention is characterized in that the oligonucleotide molecules comprise diagnostic nucleotides, which distinguish different serotypes of Salmonella. These diagnostic nucleotides are specific for a certain or a group of certain serovars. Hybridization under stringent conditions of the oligonucleotide molecules with the Salmonella genetic regions defined above is only enabled with the selected serovars and forms mismatches with other serovars which differ in their genetic code. Hybridization under stringent condition is performed for example in solution with 124 µl DEPC-treated water, 2 µl 10% SDS, 4 µl 50x Denhardt's reagent (Sigma), 60 µl 20x SSC and 10 µl target DNA or RNA including incubated at 65 °C for 1-15 min (see examples below). The optimal hybridization conditions, including temperature, can of course vary depending on the oligonucleotide molecules. Hybridization temperatures of given oligonucleotide molecules can easily be calculated with the methods described below.

Preferred diagnostic nucleotides of the oligonucleotide molecules are selected from the underlined nucleotides given in the SEQ ID NOs 1 to 193 (table 1 below). The oligonucleotide molecules according to SEQ ID NOs 1 to 193 may be aligned directly or after conversion into complementary and reverse form with the appropriate genes; since the genes may be used after amplification, e.g. by PCR, and can be available in sense and anti-sense orientation. Complementary nucleotides are A and T or U, and G and C. The examples of oligonucleotide molecules provided in table 1 comprise at least one diagnostic mismatch (also referred to as diagnostic nucleotide), i.e. a nucleotide which discriminates between certain serovars by selective hybridization of the given Salmonella serotype and forming mismatches with other serovars. Thus, according to SEQ ID NOs 1 to 193 preferred diagnostic nucleotides are for the fliC gene at nucleotides number 714, 720, 730 according to the sequence for probe c1 and for the gyrB gene at nucleotides number 753, 762, 768 according to probe g4, etc.. A nucleotide comprising such a diagnostic nucleotide, where the rest of the oligonucleotide molecule, i.e. the not underlined part of the sequences given in table 1 below, can be modified, is preferred. This modification of the oligonucleotide molecules can be performed either by selecting other nucleotides, which are comprised by the sequences of the gyrB gene, the atpD gene, the fliC gene or the fljB gene or by selecting nucleotides, which are not comprised by those sequences, thus forming non-diagnostic mismatches, which are common to all Salmonella serovars. Such non-diagnostic mismatches of course strongly influence the hybridization condition without altering the serovar specificity. The set according to the invention is preferably used on PCR enriched products of the gyrB gene, the atpD gene, the fliC gene or the fljB gene, thus gene specificity is sorted out at the PCR level. Serovar specificity is determined with the oligonucleotide set, which therefore can comprise certain liberties such as non-diagnostic mismatches without unexpected side-effects such as cross-genetic reactivity to other genes.

In another embodiment of the present invention the set is characterized in that the regions (or diagnostic regions) of the gyrB gene, the atpD gene, the fliC gene or the fljB gene are located at the 5'-end-half of these genes or the central part of these genes. For certain oligonucleotide molecules the hybridization is best performed as close as possible to the 5'-end of the gyrB gene, the atpD gene, the fliC gene or the fljB gene. Other sets are best for diagnostic region, which are as central as possible. These preferences depend highly on the use of the oligonucleotide molecules, i.e. on their fixation, e.g. onto a solid support by spacers, which in turn can vary in length and flexibility and thus influence the hybridization preferences of the oligonucleotide molecules to Salmonella genetic material.

A further embodiment provides the set according to the invention with 1 to 4 non-diagnostic mismatches. Non-diagnostic mismatches are mismatches (i.e. nucleotides that do not form base pairs in an alignment or hybridization of the oligonucleotide molecule with the respective genes) which are common to all Salmonella serovars under observation. Non-diagnostic mismatches can be used to alter the hybridization conditions, e.g. by lowering the melting temperature. However with an increasing number of non-diagnostic mismatches the analytic quality suffers increasingly. Preferably the non-diagnostic nucleotides of SEQ ID NOs 1 to 193 are targets for non-diagnostic mismatches.

A preferred set according to the invention, is characterized in that said oligonucleotide molecules are selected from SEQ ID NOs 1 to 193 (given in table 1 below). Tests with this set have shown to be highly efficient. Such high efficiency is not only characterized by a high specificity for the Salmonella gene sequences, but also by efficient binding kinetics, which is achieved by designing oligonucleotides, which avoid the formation of adverse structures such as hairpin structures.

Genetic material is often pre-amplified by molecular biological methods using transcriptases, such as PCR. In a PCR complementary strands of genetic material are created. Thus specificity can also be achieved by creating nucleotides, which bind to a complementary strand (in reversed order). Some oligonucleotides given in SEQ ID NOs 1 to 193 are specific for such a complementary strand. A further preferred set according to the invention comprises oligonucleotide molecules, which are complementary and/or in reversed order to oligonucleotides selected from the group of SEQ ID NOs 1 to 193.

Preferably, the oligonucleotide molecules comprise, preferably attached to their 5'-end, spacer molecules. When attached to a solid support these spacer molecules will prevent the negative steric effects and interference on the hybridization properties of the immobilized oligonucleotide molecules resulting from crowding of these immobilized molecules. With the spacer molecules accessibility of the immobilized oligonucleotide molecules is provided.

Still preferred said spacer molecules comprise a C-linker, preferably a C5- to C20-linker, particularly preferred this is a C12-linker. This C-linker has proven to provide an effective and functional spacer molecule without affecting the hybridization step.

Advantageously, said spacer molecules further comprise thymidine residues, preferably 2 to 10 thymidine residues. For example extra 5 thymidine residues at the 5' end are attached to the oligonucleotide molecule or to the C-linker. These thymidine residues provide additional optimal spacing.

For a quick and simple detection it is preferred that said oligonucleotide molecules are labeled or marker labeled. The person skilled in the art will be able to select the optimal label whereby preferably such labels will be used which provide a signal or change a signal when hybridization with the target occurs. These labels may be for example enzymatic or chemical labels, such as Cy5/Cy3.

According to another aspect of the present invention a solid support is provided which comprises immobilized to its surface the set of oligonucleotide molecules according to the present invention as defined above. This solid support can be easily and effectively used in high-throughput detection and quantification assays. A large amount of various solid supports have been described in the state of the art and the person skilled in the art will be able to select the most optimal depending on the amount of oligonucleotide molecules to be immobilized, the amount of samples to detect, the possibilities in a laboratory and according to other criteria. It is possible to provide a solid support having immobilized at least 4 oligonucleotide molecules of the inventive set, however, still preferred at least 20 and most preferred at least 50 oligonucleotide molecules of the inventive set are immobilized to the solid support in order to provide a tool for high-throughput analysis.

Preferably, the solid support is selected from the group consisting of a slide, membrane, column, beads and plate. These are conventional solid supports for the detection and quantification of specific targets in a sample and have been well described in the state of the art as mentioned above.

Still preferred the solid support comprising oligonucleotides according to the invention comprises a microarray of the oligonucleotide molecules according to the invention. A microarray comprises a large number of immobilized oligonucleotide molecules provided in high density on the solid support. A microarray is a highly efficient tool in order to detect hundreds to millions of target molecules in one single detection step. Such microarrays are often provided as slides or plates, in particular microtiter plates. In the state of the art a microarray is both defined either as a miniaturized arrangement of binding sites (i.e. a material, the support) or as a support comprising miniaturized binding sites (i.e. the array). Both definitions can be applied for the embodiment of the present invention. For the first of these definitions the preferred embodiment of the present invention is a miniaturized arrangement of the oligonucleotides of the present invention in a microarray. The oligonucleotide molecules are preferably immobilized onto the microarray with the help of a printing device which ensures immobilization in high density on the solid support. This microarray is particularly useful when analysing a large number of samples.

Advantageously, said oligonucleotide molecules are immobilized in spots on the surface. By providing spots, specifically defined regions comprising the immobilized oligonucleotide molecules are produced. In case one spot is specific for certain bacteria it is possible to exactly define the bacteria present in the sample analysed. It is further possible to provide a reference oligonucleotide molecule in the spots which is not only a positive control but also can be used as a reference for quantification.

Preferably, identical oligonucleotide molecules are immobilized in one spot. A positive hybridization signal of a given sample allows specific statements with respect to exactly which serotypes are present in the tested sample, since each spot will be specific for a given characteristic diagnostic region.

A further aspect of the present application relates to a method for producing an inventive solid support as defined above whereby an inventive set of oligonucleotide molecules as defined above is immobilized, preferably in spots onto the surface of said solid support. For this aspect the same definitions and advantageous embodiments apply as mentioned above for the other aspects of the present invention. With this method a solid support is provided which can be used in high-throughput analysis of any given sample.

Preferably, said set is spotted onto said surface with a single pin. By spotting with a single pin variation inherent in spotting with multiple pins is avoided. Therefore, each spot is provided with an exactly defined amount and concentration of oligonucleotide molecules.

Still preferred said spotting is carried out with a printing buffer, preferably with DMSO in H₂O. The printing buffer is used in order that the solid support does not dry out during long spotting rounds. DMSO in H₂O, preferably about 50% DMSO in H₂O, is used since this printing buffer was optimal in keeping the solid support moist compared to aqueous solutions such as 3xSSC or phosphate buffers.

Preferably the solid support comprising a set of oligonucleotides according to the invention is characterized in that it also includes spots with mismatch counterpairs to spots with oligonucleotide molecules specific for Salmonella genes. These mismatch counterpairs (also referred to as mismatch controls) comprise mismatches in their diagnostic nucleotide(s), i.e. which relate to nucleotides which are naturally different in certain Salmonella serotypes, thus having an altered serovar specificity. If the probe and its mismatch control provide the same intensity in an assay, the probe is considered negative; only if its mismatch control is significantly weaker in signal is the signal on the probe considered truly positive.

A further aspect of the present invention is a method for serotyping Salmonella in a sample characterized in that it comprises the steps of
- amplifying the *gyrB, atpD, fliC* and *fljB* genes of Salmonella in the sample.
- adding the amplified product to the solid support as defined above,
- carrying out a hybridization step for hybridizing nucleic acid molecules from the amplified product with the immobilized oligonucleotide molecules, preferably followed by at least one wash step,
- detecting hybridization products, a hybridization product showing the presence of any one of the serotype in said sample.

Again, here the same definitions and preferred embodiments as mentioned above apply. Preferably the amplification is performed by PCR.

The wash step is carried out in order to wash away any non-immobilized oligonucleotide molecules as well as any further substances used during the hybridization step which could influence the detection of hybridization products.

The detection of hybridization products is carried out according to any known method, e.g. either the immobilized oligonucleotide molecules comprise a label which sends a signal when hybridization occurs or the nucleic acid molecules from a specific Salmonella are labeled which label is then detected after the wash step. Of course, any necessary pretreatment of the sample can be carried out prior to adding said sample to the solid support, in particular treatment for breaking open bacteria walls and setting free the nucleic acid molecules of the bacteria and the amplification of the nucleic acid molecules for example with PCR.

The hybridization step is carried out under stringent hybridization conditions which allow sufficiently specific hybridization, e.g. the number of false positive results is minimized. The person skilled in the art is able to select the optimal hybridization conditions by selecting from a variety of hybridization buffers, temperatures and wash conditions. Hereby, the melting temperature Tm is useful for selecting optimal hybridization conditions. Said Tm can be calculated with the help of known formulas, as for example given in Sambrook et al., Molecular Cloning: A Laboratory Manual (Cold Spring Harbor Laboratory, New York, 1989).

Preferably all four genes selected from *gyrB, atpD, fliC* and *fljB* are PCR amplified during the process. Examples of primers for the PCR are given in table 2 below, however any primers specific for these genes can be used.

In order to improve probe specificity, both strands are used for probe design, i.e., some probes target the positive, others the negative strand of the double stranded DNA. This strategy optimizes the mismatches between probes and non-target sequences by selecting the strand and determining a stronger mismatch against non-target sequences.

The method may include a brief (4-8 hours) preenrichment of *Salmonella* from the analysed sample. The DNA of the bacteria grown in the preenrichment can then be labeled and hybridized against the microarray. The hybridization pattern enables the identification of *Salmonella* serotypes and, in many cases, also parallel identification.

Preferably, the nucleic acid molecules from a specific bacteria hybridize to oligonucleotide molecules immobilized in a defined region, preferably a defined spot, of said surface. This will occur when identical oligonucleotide molecules specific for one bacteria are immobilized in a defined region, preferably defined spot. A detected hybridization product in any given region or spot therefore will allow statements with respect to the presence or absence of specific bacteria in said sample.

Advantageously, the sample is pretreated by isolating and purifying nucleic acid molecules from said bacteria. This can be carried out according to any known protocol whereby the nucleic acid molecules must remain intact, e.g. not be submitted to any mutations or recombinations.

Still preferred the nucleic acid molecules from the bacteria or the amplified product are fragmented, preferably to a size of 30 to 70, preferably 45 to 55, nucleotides. Such fragmentation results in a significant enhancement of hybridization. Furthermore, fragmentation decreases in many cases the differences between the hybridization capacities of probes. An average fragment size of 50 nucleotides is optimal.

According to an advantageous method said sample is pretreated so that said nucleic acid molecules from said bacteria are RNA molecules. RNA molecules have the advantage that they can be fragmented in a random manner via chemical fragmentation. Furthermore, RNA molecules allow direct incorporation of label nucleotides by for example the T7 RNA polymerase which is very efficient. RNA molecules can be produced by, for example, carrying out a transcription step for example after a PCR reaction, e.g. after amplification of DNA molecules.

As mentioned above, preferably the method of serotyping Salmonella according to the invention is characterized in that the *gyrB, atpD, fliC* and *fljB* genes of Salmonella from the sample are amplified by PCR.

Advantageously, nucleic acid molecules from the Salmonella are labeled, preferably with Cy3 and Cy5, respectively. This is achieved preferably by direct incorporation of the label during polymerase reaction for example with the T7 RNA polymerase which is carried out very efficiently. Even though Cy dyes are sensitive to nucleophilic attack these dyes allow a significant increase in the signals. Furthermore, Cy3 and Cy5 dyes are well-known and have been extensively characterized in the literature. Therefore, a preferred method comprises the steps of DNA isolation from the sample, PCR, in vitro transcription including a labelling step and fragmentation of the RNA.

Preferably, the hybridization step is carried out in conditions allowing perfect match and single mismatch hybridization. The single mismatch hybridization is sufficient to provide a specific detection of any one of the bacteria in question. Such hybridization conditions can for example be achieved by providing a hybridization mix with 124 µl DEPC-treated water, 2 µl 10%SDS, 4 µl 50x Denhardt's reagent (Sigma), 60 µl 20x SSC and 10 µl target nucleic acid molecules and hybridizing at about 55°C for several hours. These hybridization conditions provide for an optimal and highly efficient method for detecting and quantifying any one of the above defined bacteria.

Still preferred the hybridization step is carried out overnight, preferably using a shaker. The overnight hybridization ensures complete hybridization, the shaker allows an optimal spreading of the sample on the solid support and in case of the production of bubbles occurring during hybridization these are moved to the edge of the solid support due to the motion of the shaker. With a shaker enough extra mixing is provided in order to allow reasonably uniform hybridization across the whole solid support. Therefore, an efficient hybridization is achieved.

Advantageously, the hybridization step is carried out in a sticky hybridization chamber. This is a self-adhesive plastic film which, when stuck onto a glass array, creates a closed hybridization "chamber", for example of about 200 µl in volume. Such a hybridization chamber allows significantly higher volumes than those which exist between a microarray and a traditional cover slip.

The set of oligonucleotide molecules of the present invention can also be used as PCR primers. Therefore another aspect of the present invention is a method for serotyping Salmonella in a sample characterized in that it comprises the steps of
- amplifying fragments of the *gyrB, atpD, fliC* and *fljB* genes of Salmonella in the sample by PCR using oligonucleotides from the set according to any one of claims 1 to 10,
- detecting PCR products, a PCR product showing the presence of one of the serotype in said sample.
PCR amplification of genetic fragments can be performed by standard methods known in the art (see Sambrook et al, above; or Ausubel et al., Current Protocols in Molecular Biology, Green Publishers Inc. and Wiley and Sons, New York (1994)). The presence of a PCR fragment indicated the hybridization of the primer with the genetic material and the subsequent amplification of a fragment of the same, thus indicating a Salmonella serovar to which the primer is reactive or specific.

The present invention is described in more detail with the help of the following examples.

### Examples

### Example 1: Oligonucleotides

Table 1. Oligonucleotides for Salmonella serovar identification. The first letter of the probe name refers to the targeted marker gene. a is for atpD, g is for gyrB, c is for fliC, b is for fljB. The fourth column shows the sequences of the probes designed according the criteria set by the methodology according to the invention. Diagnostic nucleotide positions are marked in greyscale and by underlining. The positions of the diagnostic regions are given in the fifth column according to the corresponding gene in Salmonella Typhimurium LT2 (accession nr. NC_003197.1). The oligonucleotides given below cover about 40 serovars, which account for approximately 99.9% of all cases. The third column indicates the direction as "s" for sense and "a" for anti-sense relative to the genetic sequences. Anti-sense sequences correspond to complementary and reverse sequences in sense orientation.

| **SEQ ID No.** | **Probe** | **dir** | **sequence 5'-3'** | **position** | **specificity** |
|---|---|---|---|---|---|
| 1 | c1 | s | C**AAGAC**G**ACTAAATCTACTGCTGGTAC** | 714 | Agona/ Bovismorbificans/ Mbandaka/ Senftenberg |
| 2 | c1_rev-comp | a | **GTACCAGCAGTAGATTTAGT**C**GTCTT**G | 714 | Agona/ Bovismorbificans/ Mbandaka/ Senftenberg |
| 3 | g4 | s | GGTGTGGAAGTAGCGCTACAGT | 751 | Agona/ Senftenberg/ Mbandaka |
| 4 | c2 | s | TTCAAGTTGGGGCGAATGACG | 437 | Albany |
| 5 | c3 | s | GTGGTCCCGCAGCAGATAAATTATC | 620 | Albany/ houtenae |
| 6 | g5 | a | TGCCGCCTTCGTAGTGGAAATG | 643 | all Salmonella enterica Serovars |
| 7 | c4 | a | CCTGCCGCATCGTCTTTCG | 122 | all Salmonella enterica Serovars |
| 8 | b54 | s | CAACAACAACCTGCAGCGTGTG | 255 | all Salmonella enterica serovars |
| 9 | b55 | s | GGACAACACCCTGACCATCCAG | 420 | all Salmonella enterica serovars |
| 10 | c5 | s | AACAACAACCTGCAGCGTGTG | 256 | all Salmonella enterica Serovars |
| 11 | a4 | a | GCAGTTCCTGAGAGTTTGACAACTC | 355 | all Salmonella enterica Serovars |
| 12 | b53 | s | CTGAACGAAATCGACCGTGTATCCG | 358 | all Salmonella enterica Serovars |
| 13 | b56 | s | AACGGCG**TGAA**AGTCCTGGC | 397 | all Salmonella enterica Serovars |
| 14 | b57 | s | GAACTGG**CGGT**TCAGTCTGCT | 280 | all Salmonella enterica Serovars |
| 15 | b58 | a | CAGCGCGCC**TT**CAGTGGT | 229 | all Salmonella enterica Serovars |
| 16 | b59 | a | CGCAGCG**CATC**CACCTGC | 1284 | all Salmonella enterica Serovars |
| 17 | c7 | s | GG**AA**AT**CC**CAC**G**G**CAA**CAGG | 595 | Anatum |
| 18 | a6 | s | GGC**T**A**T**T**C**ACC**G**CGCGG | 324 | arizonae |
| 19 | a7 | s | GGGCTATTCACC**G**CGCGG | 323 | arizonae |
| 20 | g7 | a | **GC**G**TC**GGAC**GGATTTT**CCAG | 1084 | arizonae |
| 21 | g6 | a | **G**T**ACCATCGT**G**CCAG**TTT**TATCGG** | 479 | arizonae |
| 22 | g8 | a | **TGTCTTTGGT**C**TG**C**GA**G**GAAAA**T**TTC** | 990 | arizonae |
| 23 | g9 | a | **TG**C**TT**T**GC**AG**ATCC**G**CCC** | 1284 | arizonae |
| 24 | g11 | a | GGCA**CGAG**C**GG**CATCAAT | 1126 | arizonae |
| 25 | c11 | a | **ATTGATCGGCTTTTAAAT**C**TGCTCCA** | 1044 | arizonae/ Blockley |
| 26 | c8 | s | AAACCGTTGACGC**A**TTCCGTAG | 1250 | arizonae/ Blockley |
| 27 | c9 | a | **GG**C**AGT**A**CCCTCCGTAGTTTTA** | 1175 | arizonae/ Blockley |
| 28 | g15 | a | CGCAACTTCAACACCGATACCG | 744 | Blockley |
| 29 | c16 | a | GGAAACCATTTTTA**CCTG**AGTCAGTCG | 668 | Blockley |
| 30 | b1 | s | CTAAGAATTCCTTAATCCGGGCGG | 896 | Blockley |
| 31 | b2 | s | CGTTGCCCGCCGATGCTA | 920 | Blockley |
| 32 | g14 | a | GAG**T****C**CCG**CC**AT**C**ACGCT | 824 | Blockley |
| 33 | g16 | a | **AGCTT**A**TC**T**TT**G**G**T**CT**GGGAGG**AG** | 996 | Blockley |
| 34 | g17 | s | **CGAA**C**TCTA**CC**T**G**GT**G**GA**A**GG**G | 1254 | Blockley |
| 35 | b5 | a | **GTGATTTCAGCCTAG**A**T**G**GAGTCGAG** | 325 | Bovismorbificans/ Goldcoast |
| 36 | c20 | s | **GCGATAGCT****A**G**TG**C**CATTAA**G**GGT** | 754 | Bovismorbificans/ Senftenberg |
| 37 | b6 | s | ACTG**C**C**GATGCAA**A**TG**C**CG** | 925 | Bovismorbificans/ Zanzibar/ Goldcoast/ Kottbus |
| 38 | c21 | s | CTGGCGACGCTACTAAAAATGGTG | 698 | Bradeney/ Brandenburg/ Braenderup |
| 39 | g18 | s | **CTAAT**T**AC**C**GC**GC**T**G**GG**CTG | 1408 | Bradeney/ Schwarzengrund |
| 40 | b9 | s | **ACCCCA**A**GT**T**ATACCGCTG**C**TG** | 1072 | Bradeney/ Schwarzengrund |
| 41 | b10 | a | GCTTTAAGCGCATAACCACCTTCA | 990 | Braenderup |
| 42 | b11 | a | **GGTTNTAGCTT**T**AACTGCTCCTGTCG** | 1049 | Braenderup |
| 43 | a9 | s | CTGATGTGCCCGTTCGCG | 406 | Braenderup/ Coeln/ Newport |
| 44 | a10 | a | CCGGTTCGCCCAGAACGT | 269 | Brandenburg |
| 45 | b12 | a | AGGTAGAACTGATAACGTCTGTCGC | 553 | Brandenburg |
| 46 | g19 | a | CACGCGCGGCATCGATAATATT | 1120 | Brandenburg/ Chester |
| 47 | a11 | a | AGCAGTGCTGATACTTCCGTACC | 754 | Cerro |
| 48 | c22 | a | ATCTAAGCCCAGCTCAGTTTTATCGA | 488 | Cerro |
| 49 | c25 | a | CCAAGTCTACGGCAGTACCCT | 1187 | Cerro |
| 50 | c27 | s | GTGATGTAAAAATTGCGGCAGCTGAT | 572 | Cerro/ Blockley/ arizonae |
| 51 | g20 | a | CGGATGGAAACGCCTGAGTTC | 591 | Cerro/ London/ Kottbus |
| 52 | c28 | a | CNATAGTAACAACCTCGGTTTTACCGTC | 1102 | Chester |
| 53 | c29 | a | TGATTTCGGATCCATTGCATCAGTTG | 548 | Chester |
| 54 | c30 | a | **C**G**AGGATGTTGGAGACTTCGGT** | 1381 | Chester |
| 55 | b14 | s | **GGATACGCTGAACGTGCAGAAAG** | 510 | Chester |
| 56 | b13 | s | **AGGTTGG**C**GCGAACGA**C**G** | 440 | Chester |
| 57 | a12 | a | **A**CAAC**TCTTC**G**TAAGA**A**GGCGCT** | 339 | Chester/ Brandenburg |
| 58 | a13 | a | **CGG**C**TG**A**TA**A**CC**T**ACTGC**G**GA**A | 789 | Chester/ Brandenburg |
| 59 | a14 | a | CGAACAGCAGTACATCACGACCT | 702 | Choleraesuis |
| 60 | g21 | a | **GTTGGTAAA**GG**TTTC**GTG**G**C**T**C**G** | 512 | Choleraesuis |
| 61 | b15 | s | CAAAAC**TT**CCATGCCT**AA**GCGAACT | 209 | Choleraesuis |
| 62 | b16 | s | CGATTTCTGA**CTGT**GTTAGCCTGG | 249 | Choleraesuis |
| 63 | b19 | s | CCCAAAGCG**T**AACCA**TG**TGTGAA | 117 | Choleraesuis |
| 64 | b26 | s | GTTATTGTGTAGCCAGAATGCCGC | 186 | Choleraesuis |
| 65 | b20 | s | AAGCGAACT**GTTG**AGAGTACGTTTC | 225 | Choleraesuis |
| 66 | b21 | s | GGAAGTGCT**TGTC**CCAACCTTG | 271 | Choleraesuis |
| 67 | b22 | s | AATTTTGAGCTAGTAGGGTTGCAGC | 142 | Choleraesuis |
| 68 | b24 | s | CAGCCACGAGTAAGTCTTCCCTTG | 163 | Choleraesuis |
| 69 | b25 | s | GAGAGATCC**CCTC**ATAATTTCCCCA | 96 | Choleraesuis |
| 70 | b28 | s | GACGGCACTAGCAAAACAGCAG | 1093 | Choleraesuis/ Infantis |
| 71 | c41 | a | CGTCAGCAGCAGTATAACTTGTGGTT | 1038 | Coeln |
| 72 | c42 | a | TTAATTGCTCCTGTCGCTTCATCG | 1008 | Coeln |
| 73 | c36 | s | GTAAAACAATTGAAGGCGGTTATGCG | 947 | Coeln |
| 74 | c37 | a | GTTGGTTAGCCGCTGTTTTGGTA | 1068 | Coeln |
| 75 | c39 | a | AAAGTTGTGACCTTCAGCTTTGCT | 1150 | Coeln |
| 76 | a31 | a | ACAACTCTTCATAGGAAGGCGCTG | 338 | Derby |
| 77 | g2 | a | TCAGCGTGGCCACTTCCT | 1394 | Derby |
| 78 | c44 | a | GATCGATTTCCTCCAGACGTTGC | 351 | Derby |
| 79 | g22 | s | CGTAACCGTAAAAACCAGGCGAT | 1306 | Derby/ Virchow/ Anatum/ Albany/ Kottbus |
| 80 | a15 | a | TTCATATCGACCGGTTCACCCAG | 274 | diarizonae |
| 81 | c47 | a | **G**CC**A**GC**GC****A**G**CATCAATTTTC** | 1230 | diarizonae |
| 82 | g25 | a | **CGGATC**G**CG**T**TCCTG**A**CAATC** | 1225 | diarizonae |
| 83 | g27 | s | **AA**A**A****T**TA**T**C**GA**C**GC**C**GCCCG** | 1120 | diarizonae |
| 84 | c46 | a | **GGCTTTACTCGCA**GC**GTA**A**GTTT** | 1136 | diarizonae |
| 85 | c48 | a | **GAATCTG**CG**CACG**A**GACATGTT** | 1393 | diarizonae |
| 86 | c50 | a | C**CGC**G**CCAATGGCAATATCA** | 903 | Dublin |
| 87 | c51 | a | **C**T**TTATCCGGTGCT**A**C**TG**CATCAGT** | 610 | Dublin |
| 88 | c52 | s | **CTGTAGT**G**AC**T**GATG**CA**GCAGC** | 602 | Enteritidis |
| 89 | g28 | a | **TCCAGCAAGTATTCGCTCAGCA** | 1067 | Enteritidis/ Dublin |
| 90 | c54 | s | **ACGCC**A**CGGGTGATAAGATCA** | 1103 | Enteritidis/ Dublin |
| 91 | b30 | a | **GACCTTTAATGTTCGCG**G**TGAAACG** | 157 | Goldcoast |
| 92 | a17 | s | **TCACCGACCTCCAGCACC** | 206 | Goldcoast |
| 93 | c55 | s | C**TCCAACATGTCCCGCG**C | 1389 | Goldcoast |
| 94 | a16 | a | **T**C**T**T**CACC**G**ATGTC**G**CCTTTCA** | 293 | Goldcoast |
| 95 | a18 | a | **GGCTTTACCTAC**C**GGAACT**T**C**G**AT** | 226 | Goldcoast |
| 96 | g30 | a | GCTGT**A**GCCTT**CTTT**GTCCATATAGG | 884 | Goldcoast |
| 97 | b32 | s | ACCAACTGCAACTGATATTAAAGCTGC | 606 | Hadar |
| 98 | c59 | a | ACAACTTCAGTTTTACCGTCTGCG | 1098 | Hadar/ Mbandaka |
| 99 | c57 | a | CGGGTTTTCGGTAGTTGTAGCAG | 1202 | Hadar/ Mbandaka |
| 100 | c58 | s | GGTGATGACTACTATGCTGCAACT | 985 | Hadar/ Mbandaka |
| 101 | a19 | a | GGAAGTGATAGAACCGGTCTTGGT | 856 | Hadar/ Zanzibar0 |
| 102 | b34 | a | CGGTACCCAGTGCGGACT | 65 | Heidelberg |
| 103 | c60 | a | CCTGGATGGTCAAGGTGTTGTC | 421 | Heidelberg/Infantis |
| 104 | a20 | a | TTCATCTGACCATACACCAGGGAT | 609 | houtenae |
| 105 | a22 | s | CGGTATCGTACGTACCATCGCG | 147 | houtenae |
| 106 | c62 | s | GCAAACGGCTCGAACTCTGG | 298 | houtenae |
| 107 | c69 | a | AAATCAGTTTGTTCCGCGACACG | 373 | houtenae |
| 108 | g31 | a | GTGGAA**AT**G**A**TC**C**TCTTTGCCGTC | 628 | houtenae |
| 109 | c63 | a | TGAAA**G**TTGCATT**C**CCGTCTTTATCT | 1125 | houtenae |
| 110 | c66 | a | **A**CGC**G**CCTTCAGT**C**GTCT | 227 | houtenae |
| 111 | c68 | a | GCGC**AT**CATC**A**ATTTT**TG**CCAGC | 1224 | houtenae |
| 112 | b35 | a | CGCCAACGCTGCATCAATTTTC | 1263 | Infantis |
| 113 | b36 | a | TGTTGGA**G**ACTTCGGTCGC**A**TAG | 1407 | Infantis |
| 114 | a23 | s | CG**AAGG**T**C**GT**GA**C**GT****C**C**TGC** | 699 | Infantis/ Newpt |
| 115 | g38 | s | **GCT**CT**GC**G**AA**G**CA**G**GG**CC | 1289 | Infantis/ Senftenberg/ Agona/ Mbandaka |
| 116 | a24 | a | T**ACTTC**C**GTACCAGCAAGGGTG** | 744 | Kedougou |
| 117 | b38 | a | TTTCTGCAC**A**TTCAG**T**GAA**T**CCAGG | 507 | Kedougou |
| 118 | a25 | a | **CC**GG**T**TT**T**G**GTTGA**G**GT**G**ATACG** | 844 | Kedougou |
| 119 | g39 | a | CAGCG**CTAC**TT**C**C**AC**AC**C** | 751 | Kedougou |
| 120 | b37 | a | **C**AG**C**C**A**C**T**TCC**GCCAG**C**T** | 1220 | Kedougou |
| 121 | c70 | a | **GCAGACCAGA**A**GACA**G**ACG**CT | 89 | Kedougou/ Goldcoast/ Kottbus/ Newport |
| 122 | c72 | a | CAATCGTAG**T**ATC**G**G**C**ATATCCTGT | 565 | Kedougou/Typhimurium |
| 123 | c73 | s | GCTACTGGTCTTGGTGGTACTGAC | 619 | Kedougou/Typhimurium |
| 124 | c71 | s | TT**AA**GCC**T**C**G**G**CTACTGGTC**TT | 609 | Kedougou/ Typhimurium |
| 125 | b41 | a | **CTTTGCTGG**C**ATT**G**TATGTTTTACCG** | 1164 | Kottbus/Manhattan |
| 126 | b42 | a | G**TAGC**A**GC**C**GCTTC**C**GCC** | 1224 | Livingstone/ Goldcoast/ Muenchen/ Mbandaka |
| 127 | a1 | s | **TC**C**GC**G**GTAGGCTA**T**CA**G**C** | 790 | Livingstone/ Infantis |
| 128 | c75 | s | **GC**TT**TGGC**G**CAGGTTGATG** | 1243 | Livingstone |
| 129 | c74 | a | **CTGCAGCGGGTTTTC**A**GT**CT**T** | 1210 | Livingstone/ Muenchen |
| 130 | c77 | a | **GGTCTG**GCC**AGAAACACGGTC** | 370 | London |
| 131 | c76 | a | **GCTACG**G**GCTGAAG**A**CAGGTT** | 1336 | London |
| 132 | c81 | a | GC**CAG**C**GGGTTTTCAGT**G**G** | 1211 | London |
| 133 | a2 | a | GG**T**T**TT**A**CC**C**ACACC**C**GCAC** | 452 | London/ Thompson |
| 134 | c82 | a | **TGTAGGCATCCTG**G**ACATTAAG**C**TT** | 514 | Manhattan/ Muenchen |
| 135 | g42 | a | AGACGGATGGAGACGCCTG | 596 | Mbandaka |
| 136 | c83 | s | AACAACCTGTCTTCTGCCCGT | 1333 | Mbandaka |
| 137 | b43 | a | CAGACCTTTAATGTTCGCGGTAAAACG | 157 | Mbandaka |
| 138 | b44 | a | GCGCAATAGAGATACCGTCGTTAGC | 202 | Mbandaka |
| 139 | b45 | s | GGATGCAAATGCCGCGAC | 930 | Mbandaka |
| 140 | b46 | s | GCGGCAGGTCAGGCAATT | 133 | Mbandaka |
| 141 | c86 | a | GCTGCATAGGTGTCATAACCCGT | 580 | Montevideo |
| 142 | c87 | s | CAGCGGGTGCCAATAAATATCGT | 599 | Montevideo |
| 143 | a32 | s | GTAAAGCTACGCTGGGCCG | 242 | Newport |
| 144 | c90 | s | GAAGTGAATGTTGATAGTGCGACAGG | 733 | Newport/ Braenderup/ Anatum |
| 145 | c94 | a | TCAGATCTATATCGATGGTTTCACCG | 456 | Newport/ Kottbus |
| 146 | a26 | s | ACGAACCGCCGGGAAACC | 632 | Oranienburg/ Brandenburg |
| 147 | c95 | s | GGCTATCGCAACTTCTATCAAAGGC | 753 | Oranienburg/ Senftenberg |
| 148 | c96 | s | TTCTAATATGTCCAAAGCGCAGATCC | 1389 | Oranienburg/ Senftenberg |
| 149 | a27 | a | TGATACGACCCAACGTGGCTTTAC | 242 | Panama |
| 150 | c97 | s | CACCGAAAACCCACTGGCT | 1212 | Panama |
| 151 | c98 | s | KGKWAAGTCTTCTTCGATGCACTGA | 660 | Panama |
| 152 | b47 | a | ACCAGAAGACAGACGCTCAATAGC | 82 | Panama |
| 153 | b48 | s | TTGCTTCAGCAGATGCTAAAAATGCC | 878 | Panama |
| 154 | b52 | s | CCGATGCTAATGCCGCTACAT | 929 | Panama/ Blockley |
| 155 | b49 | s | TCGGGTCTTACTGATGCAGCTATT | 601 | Panama/ Infantis |
| 156 | b50 | a | ATCGGCATAAGCTGTCGTTGTTAC | 556 | Panama/ Infantis |
| 157 | b51 | a | CGTACCAGTCGTACCACCCG | 635 | Panama/ Infantis |
| 158 | g43 | s | CGTGAAGGTACCGGACCCG | 972 | Panama/ Montevideo/ Oranienburg |
| 159 | c101 | a | GCGGCTTTGATAGCAGTAGCATC | 607 | Saintpaul/ Anatum/ Kottbus/ Braenderup/ Newport |
| 160 | g46 | a | GGCATCATCGCCAGTGGC | 928 | Saintpaul/ Newport |
| 161 | a28 | s | ACATGATGGAGCTCATCCGTAACA | 476 | salamae |
| 162 | g48 | a | GGTGGAGAAGTAGAATATATTCGGGTG | 709 | salamae |
| 163 | g49 | a | GTTTTATCGGTATCGCCAGTAACGG | 464 | salamae |
| 164 | g50 | a | CCACGATTTTCGCGTCAGACG | 1094 | salamae |
| 165 | c105 | s | TGGATACTGCAGGGACTTACTGG | 576 | salamae |
| 166 | c108 | s | GCAAAGCTGTGACCGGTGTTTC | 548 | salamae |
| 167 | c112 | a | GCATAACCTCCGTCAATCGTCTT | 952 | salamae |
| 168 | c113 | s | CCCAGAAAAAAGATGGTAGCTTTAGTG | 1007 | salamae |
| 169 | g47 | a | GGCAGAATCGCCTGATTCTTGC | 1313 | salamae |
| 170 | g51 | a | ATTTCACGGGCACGACGC | 1155 | salamae |
| 171 | c103 | s | AATACCACGTCTTACACCGCAAC | 1036 | salamae |
| 172 | c106 | a | GCT**A**TCT**A**CATCATA**CGC**TTTCTGCA | 524 | salamae |
| 173 | c110 | a | CCCAGTTGGTT**C**AGTGC**TA**TTTTGG | 1070 | salamae |
| 174 | c111 | a | CTGG**A**T**A**GTCAGGGTG**TT**G**T**CC**T** | 419 | salamae |
| 175 | c115 | a | **TCTGGGCTG****T**A**ACAG**T**ATCTGTACC** | 586 | Schwarzengrund |
| 176 | b61 | a | **CCCCAGT****A**AAAG**CCCCC**A**ATAG**T**AA** | 710 | Schwarzengrund |
| 177 | c116 | a | **T**C**AGCGCTCCTT**C**AG**T**GGTC** | 228 | Schwarzengrund |
| 178 | c117 | s | **T**A**C**T**GATATCGAAACTGCAATTGGCG** | 615 | Schwarzengrund |
| 179 | b62 | s | **AGTTGTTTCCGC**A**G**A**TGCTAA**AA**ATG** | 876 | Schwarzengrund/ Bradeney |
| 180 | b63 | a | TCGG**T**CGCA**TAGTCA**G**AAT**CTTCG | 1395 | Schwarzengrund/ Bradeney |
| 181 | a29 | a | **AT**C**TCGCCTTTCAT**G**TCAACCGG** | 283 | Senftenberg |
| 182 | g53 | a | ATCTTCTTTACCATCGCGCTTGTC | 619 | Senftenberg/ Goldcoast |
| 183 | a30 | a | GGTTTTACCCACACCCGCAC | 452 | Thompson/ London/ Kottbus |
| 184 | c125 | s | AAGACGATTGATGGCGGCTTC | 952 | Thompson/ Zanzibar |
| 185 | c126 | a | GGCGTTAATGCTGATCGATCCATCTT | 1016 | Thompson/ Zanzibar |
| 186 | c128 | s | GTTGGAGACGATATTTATGCTGCAACT | 982 | Thompson/ Zanzibar |
| 187 | c123 | a | GTTGGTTCAGTGCAGTTTTTGTGTTG | 1065 | Thompson/ Zanzibar |
| 188 | c127 | a | CGGTGTATTTAGTCCCTGAACTTCAGT | 826 | Thompson/ Zanzibar |
| 189 | c130 | s | CGACTTCCCCGCTTACAGGTG | 788 | Typhimurium |
| 190 | g55 | a | TGGTCTGTGAGGAAAATTTCGGGT | 986 | Typhimurium/ Livingstone/ Goldcoast |
| 191 | c134 | a | CTGGGTAATTTCAGCCTGGATAGAGT | 329 | Virchow |
| 192 | g57 | a | **GG**TT**TTT**A**CG**G**TT**A**C**G**CCC**C**TG** | 1300 | Virchow/ Derby/ Anatum/ Albany |
| 193 | a33 | a | **G**G**CC**A**TA**C**ACCA**G**GGAGACTTTA** | 603 | Virchow/ Goldcoast |

Thus the diagnostic nucleotides are for the fliC gene at nucleotides number 714, 720, 730 according to the sequence for probe c1 (SEQ ID NO 1) and for the gyrB gene at nucleotides number 753, 762, 768 according to probe g4 (SEQ ID NO 3) etc.. SEQ ID NO 59 as listed above is complementary (and reverse) - indicated as "a" for anti-sense - to the genetic sequence of the atpD gene thus the 3' end (T) fits the genomic nucleotide (A) at position 702 given above. Therefore diagnostic nucleotides according to SEQ ID NO 59 are at positions 705, 708, 711, 714, 715, 717 and 718 of the atpD gene.

### Example 2: Microarray preparation

Oligonucleotides for immobilization were custom synthesized (VBC Genomics, Vienna, Austria) with a 5' NH₂ group, followed by a C₁₂ spacer and 5 thymidines residues preceding the probe sequence. A 384 well flat bottom plate was prepared with 30 µl of 50 µM oligonucleotide solutions in 50% DMSO. Samples were spotted with an OmniGrid spotter (1 TeleChem SMP3 pin) at 50% relative humidity (using the humidity controller of the spotter), 22°C onto silylated slides (with aldehyde chemistry, Cel Associates, Houston). Arrays were always spotted in triplicate to enable a statistical correction for errors. Spotted slides were incubated overnight at room temperature at <30% relative humidity, rinsed twice in 0.2% (w/v) SDS for 2 min at room temperature with vigorous agitation to remove the unbound DNA. Slides were then rinsed twice in distilled water (dH₂O) for 2 min at room temperature with vigorous agitation, transferred into dH₂O preheated to 95-100°C for 2 min, and allowed to cool at room temperature (~5 min). Slides were treated in a freshly (immediately before use) prepared sodium borohydride solution for 5 min at room temperature to reduce free aldehydes. Preparation of the sodium borohydride solution: 0.5 g NaBH₄ was dissolved in 150 ml phosphate buffered saline (PBS; 8g NaCl, 0.2g KCl, 1.44g Na₂HPO₄, 0.24g KH₂PO₄, in 1000 ml H₂O, pH 7.4, autoclaved) then 44 ml of 100% ethanol was added to reduce bubbling. Slides were rinsed three times in 0.2% (w/v) SDS and once in dH₂O for 1 min each at room temperature. Finally, slides were dried individually using an airgun fitted with a cotton wool filter inside (to keep oil microdroplets away from the slide surface). Dried slides were stored at room temperature in the dark prior to use.

### Example 3: Target preparation

gyrB, atpD, fliC, fljB genes can be amplified using the PCR primers UP1 and UP2r (Fukushima and Kawaguchi, J Clin Mic 40(8): 2779-2785 (2002)) , atpDF (5737) and atpDR (6625) (Christensen and Olsen, FEMS Mic Lett 161: 89-96 (1998)) , FSal and rFSal (Dauga et al., J Clin Mic 36(10): 2835-2843 (1998)), FSa2 and rFSa2 (Dauga et al. (1998)) respectively. Primers for amplification and sequencing of *atpD, gyrB, fliC* and *fljB* genes are listed in table 2 below.

**Table 2. Primers for gyrB, atpD, fliC, fljB amplification**

| Primer designation | Sequence (5' - 3') | Size of product | Reference | Annealing temperature |
|---|---|---|---|---|
| **atpDF (5737) *(atpD* forward primer)** | **TAGT-TGACGTCGAATTCC CT-** | **888 bp** | **Christensen and Olsen 1998** | **55°C** |
| atpDR (6625) *(atpD* reverse primer) | GGAGACGGGTCAGTCAA GTCATC | | | |
| FSa1 *(fliC* forward primer) | CAAGTCATTAATAC(AC) A ACAGCCTGTCGC | 1500bp | Dauga et al. 1998 | 55°C |
| rFSa1 (*ff*/*C* reverse primer) | TTAACGCAGTAAAGAGA GGACGTTTTGC | | | |
| FSa2^{a} *(fljB* forward primer) | GGCACAAGTAATCAACA CTAACAGTCTGT | 1478bp | | 58°C |
| rFSa2^{b} *(fljB* reverse primer) | CGTAACAGAGACAGCAC GTT(CT)TG(CT)G | | | |
| UP1 *(gyrB* forward primer) | GAAGTCATCATGAC-CGTT CTGCA(CT)GC(ACGT)GG (ACGT)GG(ACGT)AA(AG) | 1200 bp | Fukushima and Kawaguchi 2002 | 63"C |
| UP2r *{gyrB* reverse primer) | AGCAGGGTACGGATGTG CGAGCC(AG)TC(ACGT)A C(AG)TC(ACGT)GC(AG)T C(ACGT)GTCAT | | | |

For each target, three PCR reactions of 50 µl volume each, consisting of 1x PCR buffer, 1.5mM MgC₁₂, 50nM for each four dNT-Ps, 15 pmoles of both primers, 1 ng genomic DNA or 0.1 ng cloned PCR product as template, and 1U of Taq polymerase (Gibco BRL) were performed in a Hybaid Combi Thermal Reactor TR2 using Taq DNA polymerase in accordance with the manufacturer's instructions. Amplification conditions were: 95°C for 5 min before template was added, then 32 cycles of: 1 min at 95°C, 1 min at the annealing temperature, 1 min at 72°C ; followed by a final elongation step of 10 min at 72°C. Parallel PCR products were pooled and purified using the HighPure PCR purification kit (Macherey-Nagel), according to manufacturer's instructions. Purified DNA was dissolved in ultrapure water and DNA concentration was adjusted to 50 ng/µl and stored at -20°C.

Working under RNAse free conditions, in vitro transcription was carried out as follows. 8 µl purified PCR product (50 ng/µl), 4 µl 5x T7 RNA polymerase buffer, 2 µl DTT (100 mM), 0.5 µl RNAsin (40 U/µl) (Promega), 1 µl each of ATP, CTP, GTP (10 mM), 0.5 µl UTP (10 mM), 1 µl T7 RNA polymerase (40 U/µl) (Gibco BRL) and 1 µl Cy3 or Cy5-UTP (5 mM) were added into a 1.5 ml microcentrifuge tube and incubated at 37 °C for 4 hours. Cy3 and Cy5 are optional markers for the detection later on. RNA was purified immediately using the Quiagen RNeasy kit according to manufacturer's instructions. Purified RNA was eluted into 50 µl dH₂O.

Purified RNA was fragmented by incubating with 10 mM ZnCl₂ and 20 mM Tris.Cl pH 7.4 at 60 °C for 30 min. Fragmentation was stopped by the addition of 10 mM EDTA pH 8.0 to the reaction and putting it on ice. 1 µl 40 U/µl RNAsin was added to the fragmented target. Fragmented, labeled RNA targets were stored at -20 °C.

Reference targets and artificial target mixtures for testing the quantification potential were synthesized by mixing known amounts of purified PCR products and carrying out in vitro transcription and target fragmentation as described above.

### Example 4: Hybridization

No prehybridization was done. Hybridization was carried out in a custom tailored aluminium block used as an insert for a temperature controlled Belly Dancer (Stovall Life Sciences Inc., Greensboro, NC, USA) set at maximum bending (about 10°). The hybridization block was preheated to 55 °C for at least 30 min to allow the temperature to stabilize. An Eppendorf incubator was also preheated to 65 °C. HybriWell (Grace BioLabs) stick-on hybridization chambers (200 µl in volume) were applied onto the slides containing the arrays. Assembled slides comprising the oligonucleotide molecules given in table 1 above were preheated on top of the hybridization block. For each hybridization, 124 µl DEPC-treated water, 2 µl 10% SDS, 4 µl 50x Denhardt's reagent (Sigma), 60 µl 20x SSC and 10 µl target RNA were added into a 1.5 ml Eppendorf tube and incubated at 65 °C for 1-15 min. Preheated hybridization mixtures were applied onto assembled slides via the port in the lower positions (to minimize risk of air bubbles being trapped within the chamber). Chambers were sealed with seal spots and incubated overnight at 55 °C at 30-40 rpm circulation and maximum bending.

Following hybridization, individually sticky chambers were removed and slides were immersed immediately into 2xSSC, 0.1% SDS at room temperature. Slides were washed by shaking at room temperature for 5 min in 2xSSC, 0.1% SDS; twice for 5 min in 0.2x SSC and finally for 5 min in 0.1x SSC. Slides were dried individually using an airgun with a cotton wool filter inside. Slides were stored at room temperature in the dark and scanned the same day.

### Example 5: Scanning and data analysis

Hybridized slides were scanned at 3 lines to average, 10µm resolution with a GenePix 4000A laser scanner (Axon, Foster City, Calif., USA) at wavelengths of 532 nm and 635 nm for Cy3 and Cy5, respectively. Fluorescent images were captured as multi-layer tiff images and analysed with the GenePix Pro 3.0 software (Axon). Microsoft Excel was used for statistical analysis and presentation of results.

### Example 6: Protocol optimization

One of the main problems when designing oligonucleotide microarrays is to achieve nearly identical melting temperatures for all the oligonucleotide probes on the array. One potential approach to achieving this is the use of hybridization buffers containing tertiary amine salts. Tetramethyl ammonium chloride (TMACl) or tetraethyl ammonium chloride (TEACl) have been successfully applied to enable GC-content independent hybridization on nitrocellulose or nylon membranes. The present weakness of this approach is that the thermodynamics background is missing, making it impossible to predict the effects of further factors on the behaviour of a given oligo probe. Further, the effect of tertiary amine salts on the different present and future surface chemistries is largely unknown. Thus another approach was taken by designing oligos which should have nearly identical melting temperatures in "traditional" hybridization buffers.

Many independent studies have shown that steric effects (interference of the solid support on the hybridization properties of the immobilized oligos and steric hindrance resulting from the crowding of immobilized oligos) can seriously hinder the accessibility of immobilized oligonucleotide probes. These effects are successfully mitigated by the application of spacer molecules. It was found that a C₁₂ linker and extra 5 thymidine residues at the 5' end provided optimal spacing; addition of further thymidine residues had no significant effect on the hybridization capacity (accessibility) of the oligos tested.

50% DMSO in dH₂O was selected as the printing buffer because it did not dry out during long spotting rounds (a routine spotting of 100 oligos onto 100 slides takes about 6 hours) unlike aqueous solutions, such as 3xSSC or phosphate buffers. Further, it provided uniform spots on the slides. Standard deviation in signal intensities between replicate spots was 10-15% as opposed to 20-30% for 3xSSC). Spotting was done with a single pin to avoid variations inherent in spotting with multiple pins.

Several alternative approaches were tested for target preparation. These included direct incorporation of Cy labeled dNT-Ps into dsDNA during PCR, application of a labeled PCR primer for the generation of dsDNA targets and application of a labeled PCR primer and a biotinylated one for the generation of ssDNA targets via subsequent separation of the two strands using streptavidin coated magnetic beads. As the secondary structure of the target plays a very significant role in determining the maximal hybridization signal obtainable with a given probe ("hybridization capacity"), it was an absolute necessity to fragment the target before hybridization. As RNA can be fragmented in a random manner via chemical fragmentation, it was decided to use RNA targets. A further advantage of using RNA targets is that the direct incorporation of the Cy labeled nucleotides by the T7 RNA polymerase is very efficient. Only a four-fold decrease was observed in RNA yield when 50% of UTP was replaced with Cy3-UTP during in vitro transcription. The achieved yields of target preparation are in the range of 50 ng/µl concentration (in 50 µl final volume, starting from 8 µl 50 ng/µl template) with every 10^{th} - 12^{th} nucleotide being labeled.

Fragmentation of the target RNA to an average fragment size of 50 nt resulted in a significant enhancement of hybridization. In spite of the sensitivity of the Cy dyes to nucleophilic attack (like alkaline treatment with divalent cations used to fragment RNA), an increase by over an order of magnitude in the Cy3, as well as in the Cy5, signals was observed. Furthermore, fragmentation decreased in many cases the differences between the hybridization capacities of probes.

Cy3 and Cy5 dyes were used because of the fairly extensive literature available on the behaviour of these dyes. It was shown that Cy5 did undergo significant photobleaching during the scanning process. Thus all scanning procedures started were run to completion in order to exclude selective photobleaching of only some of the Cy5 signals on the array.

Hybridization conditions were chosen which were relaxed enough to enable the hybridization of both perfect match (PM) and single mismatch (1MM) targets. As the hybridization between oligonucleotides and any other nucleotide is a reversible process, all hybridizations were carried out overnight to ensure complete hybridization. In microarray hybridizations it is often the problem that the hybridization solution does not move and diffusion is the only process which provides some mixing. Statistical errors in the final signal are inherent in such a diffusion limited system. Further, such circumstances usually result in the edges of probe sets hybridizing more efficiently thus yielding a relatively strong signal for the edge of the spot and a much weaker one for the middle. To overcome these limitations a custom modified BellyDancer laboratory shaker and sticky hybridization chambers with significantly higher volumes than those which exist between a microarray and a traditional covers-lip, were used. The tiny bubbles unavoidably formed within this chamber were slowly moved to the edge due to the motion of the BellyDancer, and this provided enough extra mixing to provide a reasonably uniform hybridization across the whole microarray.

Standard deviation of results for (triplicate) spots of individual probes was 3% to 30% between parallel slides hybridized with targets prepared in parallel.

### Example 7: General outlines for probe set design and validation for the present invention

The most critical step of the probe design process for practical optimization is to fine tune the probe set in a way that all probes in the set display hybridization behaviour as identical as possible. In the first stage, an attempt was made to design oligonucleotides with predicted melting temperatures (according to the nearest neighbour model) of 60±2 °C. However, present models can only predict melting temperatures in solution.

The hybridization behaviour of oligonucleotide probes immobilized onto a solid surface depends on several factors:
(i) Length, GC content and exact sequence of the probe. These together are considered when predicting Tm for the oligos by using the nearest neighbour method.
(ii) Position of GC and AT pairs. The middle of the probe is more important in stabilizing hybridization. A probe where the middle contains all Gs and Cs binds its target more strongly than another with homogenous GC distribution (but with identical length and GC content).
(iii) Secondary structures of the probe and of the corresponding target. When any of these two are of significant strength, compared to the strength of hybridization between the probe and the target, a significant drop in hybridization efficiency is expected.
(iv) The exact nature of the overhanging nucleotides on the target (nucleotides immediately next to the area targeted by the probe). This comes from the nearest neighbour model, but isn't normally accounted for because the overhangs of the target sequence are not considered.
(v) Number and type of mismatches. Some mismatches have little while others have very strong destabilizing effect.
(vi) Position of mismatches. Mismatches in the middle are more destabilizing than mismatches at end positions.
(vii) Factors arising from the immobilized nature of the probes. Steric effects can hinder the formation of hybrids between the target and the bound probe. This effect is much stronger for the immobilized end of the probe. Thus, the bound end of the probe plays a lesser role in the hybridization than the free end. This applies for points 2 and 6.
(viii) Hybridization between DNA oligos and RNA fragments, as in the present case, has slightly different thermodynamics to that of DNA-DNA hybridization.

Based on the above criteria, on initial results from testing the hybridization behaviour of our probes and on published data a set of simple rules was generated which significantly improved the prediction of the hybridization behaviour of the probes. These rules are:
(i) U->C changes in the target sequence lead to an rG-dT bond which is almost as strong as the original rA-dT bond (for perfect match cases). These are not considered as mismatches.
(ii) G->A changes in the target sequence lead to an rU-dG bond which is almost as strong as the original rC-dG bond (for perfect match cases). These mismatches are considered only if they are present together with other types of mismatches.
(iii) Mismatches in the end positions are not considered. Mismatches adjacent to the end positions are considered only if they are present together with other types of mismatches.
(iv) If most of the GC content of a probe is close to the 5' (immobilized) end, the probe will display a significantly lower melting temperature than originally predicted. If the 3' nucleotide is a G or C, it is considered the same way.
(v) Probes with strong hairpin structures (deltaG >= 2.0) were considered as having an extra mismatch to the target.
(vi) High GC content probes shorter than 17 nucleotides in length display unreliable hybridization behaviour under the experimental conditions applied.

## Claims

1. Set of oligonucleotide molecules for Salmonella serotyping, **characterized in that** at least one oligonucleotide molecule is specific for the *gyrB* gene, at least one oligonucleotide molecule is specific for the *atpD* gene, at least one oligonucleotide molecule is specific for the *fliC* gene and at least one oligonucleotide molecule is specific for the *fljB* gene.

2. The set according to claim 1, **characterized in that** the oligonucleotide molecules are specific for up to 30 nucleotide long regions of the gyrB gene, the atpD gene, the fliC gene or the fljB gene.

3. The set according to claim 1 or 2, **characterized in that** the oligonucleotide molecules correspond to genetic sequences of the gyrB gene, the atpD gene, the fliC gene or the fljB gene located at the positions of these genes given for SEQ ID NOs 1 to 193.

4. The set according to any one of claims 1 to 3, **characterized in that** the oligonucleotide molecules comprise diagnostic nucleotides, which distinguish different serotypes of Salmonella, preferably selected from positions of the underlined nucleotides given in SEQ ID NOs 1 to 193.

5. The set according to any one of claims 2 to 4, **characterized in that** specific regions of the gyrB gene, the atpD gene, the fliC gene or the fljB gene are located at the 5'-end-half of these genes or the central part of these genes.

6. The set according to any one of claims 1 to 5, **characterized in that** it comprises 1 to 4 non-diagnostic mismatches.

7. The set according to any one of claims 1 to 5, **characterized in that** said oligonucleotide molecules are selected from the group of SEQ ID NOs 1 to 193 or are complementary and/or in reversed order to oligonucleotides selected from the group of SEQ ID NOs 1 to 193.

8. The set according to any one of claims 1 or 7, **characterized in that** said oligonucleotide molecules comprise, preferably attached to their 5'-end, spacer molecules, preferably a C-linker, more preferred a C5- to C20-linker, in particular a linker further comprising thymidine residues, preferably 2 to 10 thymidine residues.

9. The set according to any one of claims 1 to 8, **characterized in that** said oligonucleotide molecules are marker labeled.

10. Solid support, **characterized in that** it comprises immobilized to its surface the set of oligonucleotide molecules according to any one of claims 1 to 9.

11. The solid support according to claim 10, **characterized in that** it is selected from the group consisting of a slide, membrane, column, beads and plate.

12. The solid support according to claim 10 or 11, **characterized in that** it comprises a microarray of the oligonucleotide molecules.

13. The solid support according to any one of claims 10 to 12, **characterized in that** said oligonucleotide molecules are immobilized in spots on the surface, preferably wherein identical oligonucleotide molecules are immobilized in one spot.

14. The solid support according to claim 10 or 13, **characterized in that** it also includes spots with mismatch counterpairs to spots with oligonucleotide molecules specific for Salmonella genes.

15. Method for producing a solid support according to any one of claims 10 to 14, **characterized in that** a set of oligonucleotide molecules according to any one of claims 1 to 9 is immobilized, preferably in spots, onto the surface of said solid support, most preferred with a printing buffer, which is preferably DMSO in H₂O.

16. The method according to claim 15, **characterized in that** said set is spotted onto said surface with a single pin,

17. Method for serotyping Salmonella in a sample **characterized in that** it comprises the steps of
- amplifying the *gyrB, atpD, fliC* and *fljB* genes of Salmonella in the sample.
- adding the amplified product to the solid support according to any one of claims 10 to 14,
- carrying out a hybridization step for hybridizing nucleic acid molecules from the amplified product with the immobilized oligonucleotide molecules, preferably followed by at least one wash step,
- detecting hybridization products, a hybridization product showing the presence of any one of the serotype in said sample.

18. The method according to claim 17, **characterized in that** the amplification is by PCR.

19. The method according to claim 17 or 18, **characterized in that** nucleic acid molecules from a specific Salmonella serotype hybridize to oligonucleotide molecules immobilized in a defined region, preferably a defined spot, of said surface.

20. The method according to any one of claims 17 to 19, **characterized in that** the sample is pretreated by isolating, purifying and/or amplifying nucleic acid molecules from Salmonella.

21. The method according to any one of claims 17 to 19, **characterized in that** the nucleic acid molecules of Salmonella or the amplified product are fragmented, preferably to a size of 30 to 70, preferably 45 to 55, nucleotides.

22. The method according to any one of claims 17 to 21, **characterized in that** said sample comprises RNA molecules of Salmonella.

23. The method according to any one of claims 17 to 22, **characterized in that** the nucleic acid molecules of Salmonella are labeled, preferably with Cy3 and Cy5, respectively.

24. The method according to any one of claims 17 to 23, **characterized in that** the hybridization step is carried out in conditions allowing perfect match and single mismatch hybridization, preferably in a sticky hybridization chamber.

25. A method for serotyping Salmonella in a sample **characterized in that** it comprises the steps of
- amplifying fragments of the *gyrB, atpD, fliC* and *fljB* genes of Salmonella in the sample by PCR using a oligonucleotides from the set according to any one of claims 1 to 9,
- detecting PCR products, a PCR product showing the presence of one of the serotype in said sample.
